# EUROPEAN PATENT SPECIFICATION

(11) **EP 0 934 024 B1**
(45) Date of publication and mention of the grant of the patent: **30.08.2006**
(21) Application number: 97943488.3
(22) Date of filing: 19.09.1997
(51) Int. Cl.: A61B 17/068

(54) **COIL FASTENER APPLIER**
CHIRURGISCHE VORRICHTUNG ZUM ANBRINGEN VON SPIRALKLAMMERN
DISPOSITIF D'APPLICATION D'AGRAFES A SPIRALES

(30) Priority: 20.09.1996 US 717492; 18.09.1997 US 933294
(43) Date of publication of application: 11.08.1999
(73) Proprietor: United States Surgical Corporation, Norwalk, Connecticut 06856 (US)
(72) Inventor: HOLSTEN, Henry, E., Wolcott, CT 06716 (US); SAVAGE, Robert, C., Stratford, CT 06497 (US); MARKUS, Richard, L., Milford, CT 06460 (US)
(74) Representative: HOFFMANN EITLE
(86) International application number: PCT/US1997/016990
(87) International publication number: WO 1998/011814

(56) References cited:
- WO-A-96/03925
- US-A- 4 448 194
- US-A- 4 643 190
- US-A- 4 850 355
- US-A- 5 306 281
- US-A- 5 465 895

## Description

This application is a continuation-in-part of U.S. Application Serial No. O8/717,492, filed September 20, 1996, entitled Coil Fastener Applier.

### BACKGROUND

### 1. Technical Field

This disclosure relates generally to surgical apparatus for fastening objects to body tissue and, more particularly, to a coil fastener applier configured to apply helical coil fasteners to surgical mesh and tissue during surgical repair of the body tissue in procedures such as hernia repair

### 2. Background of Related Art

WO-A-9 603 925 discloses the features in the preamble of claim 1.

Various surgical procedures require instruments capable of applying fasteners to tissue to form tissue connections or to secure objects to tissue. For example, during hernia repair it is often desirable to fasten a mesh to body tissue. In certain hernias, such as direct or indirect inguinal hernias, a part of the intestine protrudes through a defect in the support abdominal wall to form a hernial sac. The defect may be repaired using an open surgery procedure in which a relatively large incision is made and the hernia is closed off outside the abdominal wall by suturing. The mesh is attached with sutures over the opening to provide reinforcement.

Less invasive surgical procedures are currently available to repair a hernia. In laparoscopic procedures surgery is performed in the abdomen through a small incision while in endoscopic procedures, surgery is performed through narrow endoscopic tubes or cannulas inserted through small incisions in the body. Laparoscopic and endoscopic procedures generally require long and narrow instruments capable of reaching deep within the body and configured to seal with the incision or tube they are inserted through. Additionally, the instruments must be capable of being actuated remotely, that is, from outside the body.

Currently endoscopic techniques for hernia repair utilize fasteners, such as, surgical staples or clips, to secure the mesh to the tissue to provide reinforcement to the repair and structure for encouraging tissue ingrowth. The staples or clips need to be compressed against the tissue and mesh to secure the two together.

One other type of fastener suited for use in affixing mesh to tissue, during procedures such as hernia repair, is a coil fastener having a helically coiled body portion terminating in a tissue penetrating tip. An example of this type of fastener is disclosed in U.S. Patent No. 5,258,000.

Thus, there exists a need for an improved applier device for applying coil type fasteners to tissue. Since it is often desirable to remove previously positioned or partially inserted coil fasteners from tissue, it would be advantageous if such device enabled removal of the coil type fasteners from tissue if desired. There also exists a need for an applier device which can reengage a coil fastener previously applied to tissue and drive the coil fastener further into the tissue.

### SUMMARY

There is provided a surgical coil fastener applier for use in applying coil fasteners to tissue and, in particular, for use in surgical procedures such as hernia repair to affix surgical mesh to tissue. The coil fastener applier includes a housing having a handle extending therefrom and a trigger pivotally mounted on the housing. An elongated tubular portion extends from the housing and includes a drive rod rotatably supported therein. The drive rod is provided to drive the coil fasteners into tissue. The drive rod slidably supports a plurality of coil fasteners thereon and includes a slot to engage tangs of the coil fasteners to rotate the fasteners within a sleeve. Preferably a spring insert is permanently affixed within the sleeve to guide the coil fastener distally as the drive rod is rotated.

A drive assembly is provided within the housing to rotate the drive rod in a first direction. The drive assembly includes intermeshed first and second bevelled gears oriented perpendicular to each other. The first bevelled gear engages and rotates the drive rod while the second bevelled gear receives rotary motion from an actuation assembly. Preferably, the drive assembly includes an anti-reverse mechanism to allow rotation of the drive rod in only one direction, i.e. the first direction. The anti-reverse mechanism is formed by providing a gap between the second beveled gear and a hub provided to rotate the second beveled gear. A plurality of roller pins are provided within the gap and move between reduced cross-sectional areas of the gap to engage the hub with the second beveled gear and areas of increased cross-sectional areas to release the second beveled gear from the hub. This arrangement forms a roller clutch to prevent rotation of the second beveled gear in an opposite direction thereby preventing reversal of the rotation of the drive rod and withdrawal of a coil fastener partially driven into tissue.

The actuation assembly is provided within the housing to convert the pivotal motion of the trigger into rotary motion for supply to the drive assembly. The actuation assembly generally includes a ratchet-plate gear and an idler gear. The ratchet-plate gear engages the trigger and is rotated thereby. The ratchet-plate gear rotates the idler gear which in turn rotates a gear attached to the hub. The idler gear includes stops which cooperate with a blocking member formed on the housing to limit the degree of rotation imparted to the gear on the hub. This ensures that only one coil fastener at a time is driven from elongated tubular portion during a single cycling or depression of the trigger.

The drive assembly further includes a ratchet and pawl mechanism to prevent a partial cycling of the coil fastener applier. Ratchet teeth on the ratchet-plate gear engage a pawl on the housing preventing return of the trigger to an initial start position before a complete cycle or depression of the trigger has occurred. This prevents a coil fastener from being only partially rotated out of the elongated tubular portion and thus only partially rotated into tissue.

Also provided are coil fasteners for use with the coil fastener applier. In one embodiment the coil fastener is formed with a helical coil body portion having a single tissue penetrating point at an end thereof. A straight tang is formed at the opposite end of the body portion and extends inwardly across the center of a circle formed by the coils. The tang is provided to engage a slot in the drive rod and allow the coil fastener to be rotated by the drive rod.

In an alternate embodiment, the coil fastener is formed with a straight backspan and helical coil body portions extending from each end of the back span. A tissue penetrating point is provided at a free end of each body portion. The backspan engages a slot extending completely through a drive rod and is slidably supported therein. Rotation of the drive rod rotates the coil fastener within the sleeve and into tissue.

In a further alternative embodiment, the coil fastener is formed with a straight backspan having a straight leg extending from each end of the backspan and which are parallel to each other. A semi-circular tissue penetrating portion terminating in a tissue penetrating point extends from a free end of each leg. The semi-circular tissue penetrating portions are in a common plane which is generally parallel to the backspan. The backspan of the coil fastener also engages a completely slotted drive rod and is rotated thereby.

There is also provided a surgical coil fastener applier which is configured to remove coil fasteners from tissue. The coil fastener applier has an elongated tubular portion with a coiled spring affixed therein which forms a helical inner surface and which terminates in a distalmost tip. The coil fastener applier is configured to engage a tang of the coil fastener when positioned over a coil fastener. The distal end of the coil fastener applier is dimensioned such that the distance between the distalmost tip of the elongated tubular portion and a distalmost end of a drive rod is at least half the predetermined wire diameter of a coil fastener. In this manner, upon positioning the distal end of the coil fastener applier over a coil fastener positioned in tissue, the tang of the coil fastener will be engaged by a slot in the drive rod. Specifically, the slot in the drive rod engages at least half the diameter of the tang. Further, the angle between the distalmost tip of the helical surface and the slot when the trigger is in an unfired position is approximately 110°.

The coil fastener applier for use in removing coil fasteners further includes a lockout mechanism which is engagable to immobilize the drive rod relative to the tubular portion allowing the entire instrument to be rotated to remove the coil fastener. Preferably, the lockout mechanism includes a lockout button movably mounted on the housing and a blocking member which is movable into engagement of the drive assembly to immobilize the drive rod. The blocking member preferably is a leaf spring affixed within the housing and having a hook engagable with a gear assembly of the drive assembly. The lockout button is provided with a cam to force the hook into engagement with teeth on the gear of the drive assembly.

Alternatively, a portion of the trigger may be configured to engage the leaf spring and pivot the leaf spring about a pivot point to engage the hook with the teeth on the drive assembly as the trigger is fully depressed during a firing cycle.

There is also disclosed a method of removing a coil fastener from tissue which includes providing a coil fastener applier having an elongated tubular portion with a helical inner surface and a drive rod rotatably mounted within the tubular portion. The drive rod has a slot for engaging the tang and the coil fastener. The method further includes the steps of immobilizing the drive relative to the tubular portion, engaging the coil fastener with the drive rod, and rotating the coil fastener applier to remove the coil fastener from tissue.

In another embodiment of a coil fastener applier, a threaded surface is roll formed in an interior surface of the elongated tubular portion. The roll formed threaded surface replaces the coil spring of previous embodiments and serves to thread coil fasteners out of the elongated tubular portion. Additionally, a ratchet plate clutch mechanism is substituted for the roller clutch of previous embodiments. Further, additional structure is provided on the ratchet plate gear to engage and block the second bevelled gear from rotating when the applier is in the unfired position.

### BRIEF DESCRIPTION OF THE DRAWINGS

A preferred embodiment of a helical coil fastener applier is described below with reference to the drawings wherein:
FIG. 1 is a perspective view of a preferred embodiment of a coil fastener applier;
FIG. 2 is a perspective view of a helical coil fastener utilized with the coil fastener applier of FIG. 1;
FIG. 3 is a perspective view of a distal portion of a drive rod and a helical coil fastener;
FIG. 4 is a perspective view of the distal portion of the drive rod with a plurality of helical coil fasteners loaded thereon;
FIG. 5 is a perspective view, with parts separated, of an elongated tubular portion, a spring and the drive rod with a plurality of helical coil fasteners loaded on the distal portion thereof;
FIG. 6 is a sectional view of a distal portion of the elongated tubular portion with the spring installed therein;
FIG. 7 is a perspective view of the elongated tubular portion with the drive rod inserted therein;
FIG. 8 is a perspective view, with parts separated, of a housing portion of the coil fastener applier of FIG. 1;
FIG. 9 is a perspective view of the housing portion of the coil fastener applier of FIG. 1 with the housing half removed;
FIG. 10 is a perspective view of a housing half illustrating the positioning of the idler gear and the ratchet-plate gear;
FIG. 11 is a perspective view of the assembled idler gear and ratchet-plate gear;
FIG. 12 is a sectional view taken along line 12-12 of FIG. 8 and illustrating a roller clutch mechanism;
FIG. 13 is a side view, with a housing half removed, of the housing portion of the coil fastener applier in an initial position;
FIG. 14 is a partial side view, showing the positioning of the idler gear and ratchet-plate gear in an initial position corresponding to FIG. 13;
FIG. 15 is a perspective view, partially shown in cross-section, of a distal end portion of the coil fastener applier corresponding to FIG. 13;
FIG. 16 is a view similar to FIG. 13 showing initial actuation of the coil fastener applier;
FIG. 17 is a view similar to FIG. 14 and corresponding to the position of FIG. 16;
FIG. 18 is a sectional view of the roller clutch corresponding to FIG. 16;
FIG. 19 is a perspective view, partially shown in cross-section, illustrating a helical coil fastener being driven out of the distal end of the coil fastener applier;
FIG. 20 is a side view of the housing portion, with a housing half removed, illustrating initial release of a trigger and positioning of the idler gear and the ratchet-plate gear;
FIG. 21 is a sectional view of the roller clutch corresponding to FIG. 20;
FIG. 22 is a partial side view showing the positioning of the idler gear and ratchet-plate gear along with a pawl corresponding to the position of FIG. 20;
FIG. 23 is a view similar to FIG. 22 after complete release of the trigger;
FIG. 24 is a perspective view showing the use of the coil fastener applier in the patient;
FIG. 25 is a perspective view of a tissue section and a surgical mesh secured to the tissue section by a plurality of helical coil fasteners;
FIG. 26 is a perspective view of an alternate coil fastener;
FIG. 27 is a perspective view of another alternate coil fastener;
FIG. 28 is an end view of the distal end of an alternate embodiment of a coil fastener applier;
FIG. 29 is a sectional view of the distal end of the coil fastener applier of FIG. 28 taken along line 29-29 of FIG. 28;
FIG. 30 is an isolated view of FIG. 29 illustrating the distal end of the elongated tubular portion relative to the distal end of a coiled spring affixed therein;
FIG. 31 is a view of the proximal portion of a coil fastener applier of FIG. 28 including a lock mechanism;
FIG. 32 is a partial side view of the embodiment of FIG. 31 with the lock mechanism in an engaged condition;
FIG. 33 is a sectional view of the distal end being advanced toward a coil fastener applied to tissue;
FIG. 34 is a view similar to FIG. 33 and illustrating the distal end of the fastener applier engaged with the coil fastener;
FIG. 35 is a perspective view of a patient and rotation of the coil fastener applier to withdraw the coil fastener;
FIG. 36 is a sectional view of the distal end of the fastener applier with the coil fastener partially removed from the tissue;
FIG. 37 is a side view of the coil fastener applier of FIG. 31 illustrating an alternate method of engaging the lock mechanism;
FIG. 38 is a perspective view of an alternate embodiment of a coil fastener applier;
FIG. 38A is an enlarged perspective view of the distal end of the embodiment of FIG. 38;
FIG. 39 is a perspective view of an elongated tubular portion and a drive rod having a plurality of fasteners supported thereon;
FIG. 40 is a sectional view of a distal end portion of the elongated tubular portion of FIG. 39;
FIG. 41 is a perspective view, with parts separated, of a housing portion of the coil fastener applier of FIG. 38;
FIG. 42 is a plan view of a first ratchet plate;
FIG. 43 is a sectional view taken along line 43-43 of FIG. 42;
FIG. 44 is a plan view of a second ratchet plate;
FIG. 45 is a sectional view taken along line 45-45 of FIG. 44;
FIG. 46 is a perspective view of a second bevelled gear with a ratchet plate clutch positioned thereon;
FIG. 47 is a perspective view of the ratchet plate clutch including the first and second ratchet plates;
FIG. 48 is a perspective view of an assembled idler gear, ratchet plate gear and trigger gear;
FIG. 49 is an end view of the distal end of the coil fastener applier of FIG. 38;
FIG. 50 is a view partially shown in section of the distal end of the coil fastener applier taken along line 50-50 of FIG. 49;
FIG. 51 is a view, partially shown in section of the housing portion of the embodiment of FIG. 38 and illustrating an anti-reverse leaf spring engaged with teeth on the second bevelled gear;
FIG. 52 is a view similar to FIG. 51 and showing initial actuation of the embodiment of FIG. 51;
FIG. 53 is a side elevational view showing the first and second ratchet plates of the ratchet plate clutch in an engaged condition;
FIG. 54 is a perspective view, partially shown in cross-section, illustrating a helical coil fastener being driven out of the distal end of the coil fastener applier of FIG. 38;
FIG. 55 is a view similar to FIG. 51 illustrating release of a trigger of the housing portion;
FIG. 56 is a side elevational view of the ratchet plate clutch illustrating the first and second ratchet plate in slipping engagement; and
FIG. 57 is a partial side view illustrating a stud on the ratchet plate gear engaged in a notch on the second bevelled gear.

### DETAILED DESCRIPTION OF PREFERRED EMBODIMENTS

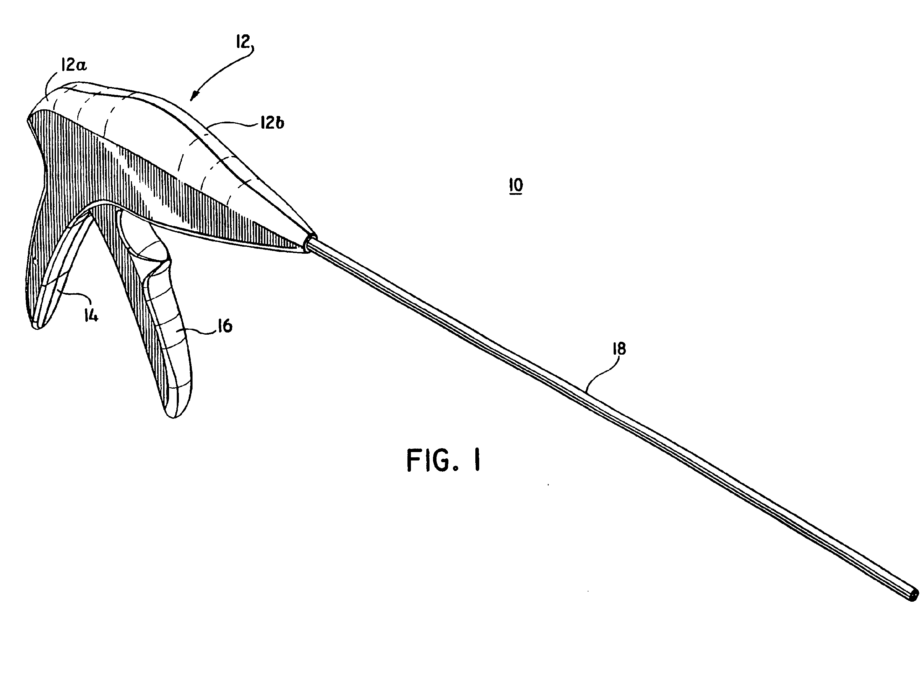

With reference now to the drawings wherein like numerals represent like elements throughout the several views and initially with respect to FIG. 1, there is disclosed a preferred embodiment of a coil fastener applier 10. Coil fastener applier 10 is provided to apply helical-shaped coil fasteners to tissue or to secure mesh to tissue during surgical procedures such as hernia repair. Coil fastener applier 10 generally includes a housing 12 which may be formed as two separate housing halves 12a and 12b and a handle portion 14 extending from housing 12. A trigger 16 is movably mounted to housing 12. Preferably, trigger 16 is pivotally connected to housing 12 with a free end of trigger 16 spaced from a free end of handle portion 14. This arrangement provides an ergonomic advantage and positive secure control of trigger 16 and coil fastener applier 10. Coil fastener applier 10 also includes an elongated tubular portion 18 extending distally from housing 12. The elongated tubular portion 18 is provided to retain a plurality of coil fasteners for application to body tissue. Elongated tubular portion 18 is preferably dimensioned to fit through conventional cannula structure. As used herein the term "distal" refers to that portion of the applier, or component thereof, further from the user while the term "proximal" refers to that portion of the applier or component thereof, closer to the user.

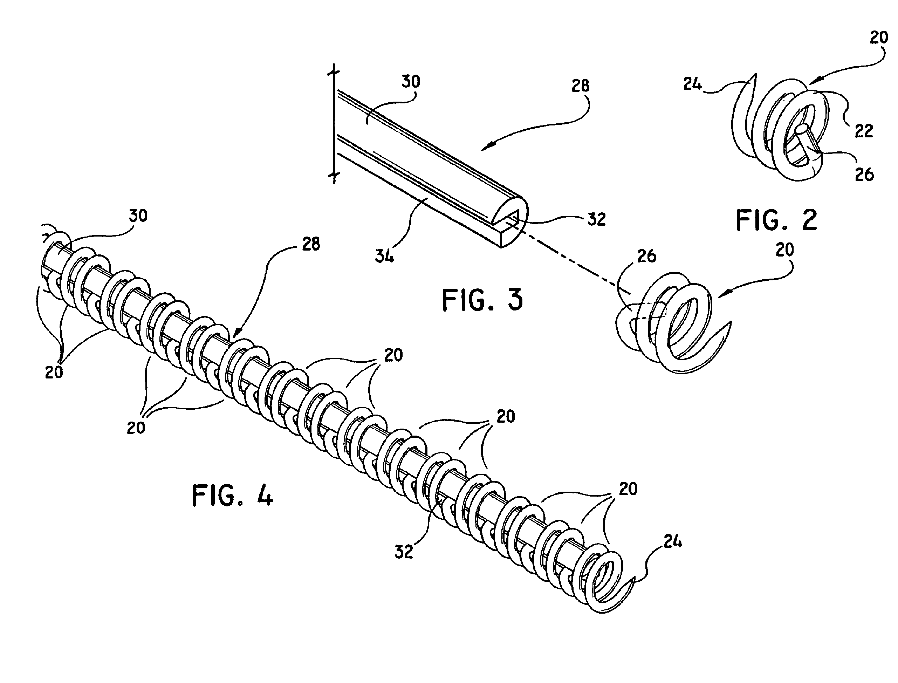

Referring now to FIG. 2, there is illustrated a helical-shaped coil fastener suitable for use with coil fastener applier 10. Coil fastener 20 is designed to be applied to tissue by rotating the coil into and through the tissue. Coil fastener 20 generally includes a coil body portion 22, preferably having approximately 2 1/2 coils and terminating in a sharp tissue penetrating point 24. A tang 26 is provided at an opposite end of coil body portion 22. Tang 26 extends generally inwardly toward the center of coil body portion 22 as shown. Coil fastener 20 is preferably formed of a suitable biocompatible material, such as, for example, titanium. However, coil fastener 20 may alternatively be formed of various elastomeric or polymeric materials and in addition may be formed of various bioabsorbable or biodegradable materials.

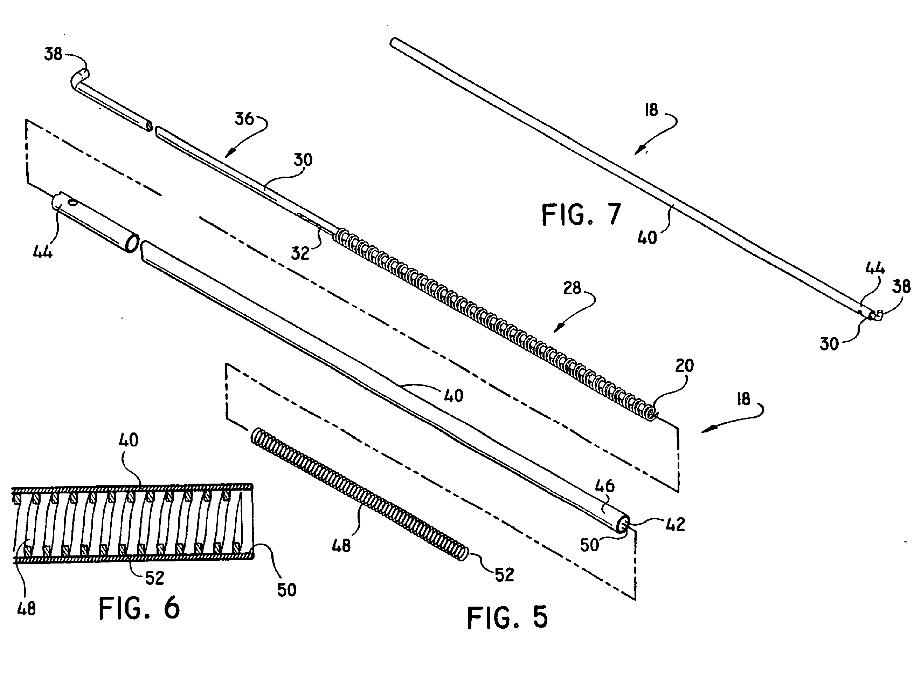

Referring now to FIG. 3, a distal portion 28 of a drive rod 30 associated with coil fastener applier 10 is provided to retain and drive coil fasteners 20. Distal portion 28 generally includes a longitudinally extending slot 32 extending along the length of distal portion 28. Slot 32 is provided to receive tang 26 therein such that upon rotation of drive rod 30 coil fastener 20 is similarly rotated. While slot 32 is illustrated as extending partially across drive rod 30, slot 32 may be formed completely through drive rod 30 to accommodate other types of coil or rotatable fasteners. A flat 34 extends adjacent slot 32 in distal portion 28.

As best shown in FIG. 4, a plurality of coil fasteners 20 may be arranged in a series longitudinally along the length of distal portion 28 of drive rod 30. Each coil fastener 20 has its associated tang 26 positioned within slot 32 of drive rod 30.

Referring now to FIG. 5, and as noted above, elongated tubular portion 18 contains a plurality of coil fasteners 20 and structure to drive coil fasteners 20 into tissue. A proximal portion 36 of drive rod 30 is of a generally solid circular cross-section such that slot 32 stops distally of proximal portion 36. A bent or L-shaped proximal end 38 of drive rod 30 is provided to assist in rotating drive rod 30 to advance coil fasteners 20 through elongated tubular portion 18 and drive coil fasteners 20 into tissue. Elongated tubular portion 18 also includes a generally tubular sleeve 40 defining a bore 42 therethrough and having a proximal end 44 and a distal end 46. Drive rod 30 is freely rotatable within bore 42 of tubular sleeve 40.

As best shown in FIGS. 5 and 6, in order to move successive coil fasteners 20 in a distal direction upon rotation of drive rod 30 there is provided a coil spring 48 which is preferably braised or welded to an inner surface 50 of tubular sleeve 40. Coil spring 48 creates a helical longitudinally extending surface 52 configured for engagement with the coil body portions 22 of coil fasteners 20. Thus, upon rotation of drive rod 30 coil fasteners 20 are moved along surface 52 and through tubular sleeve 40.

As best seen in FIG. 7, when assembled, L-shaped proximal end 38 of drive rod 30 extends out of proximal end 44 of tubular sleeve 40 for engagement with a drive assembly described hereinbelow.

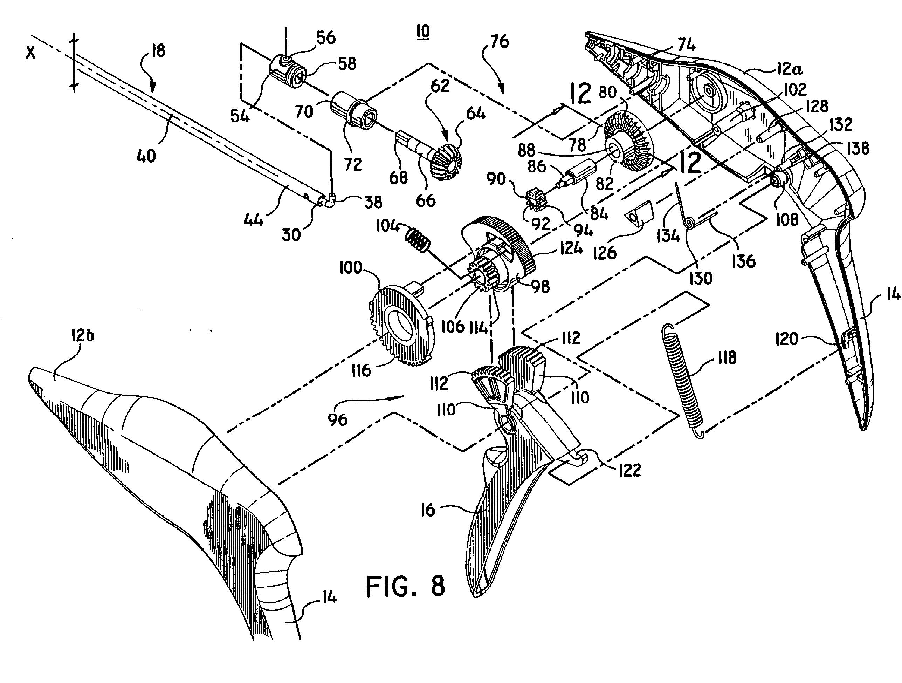

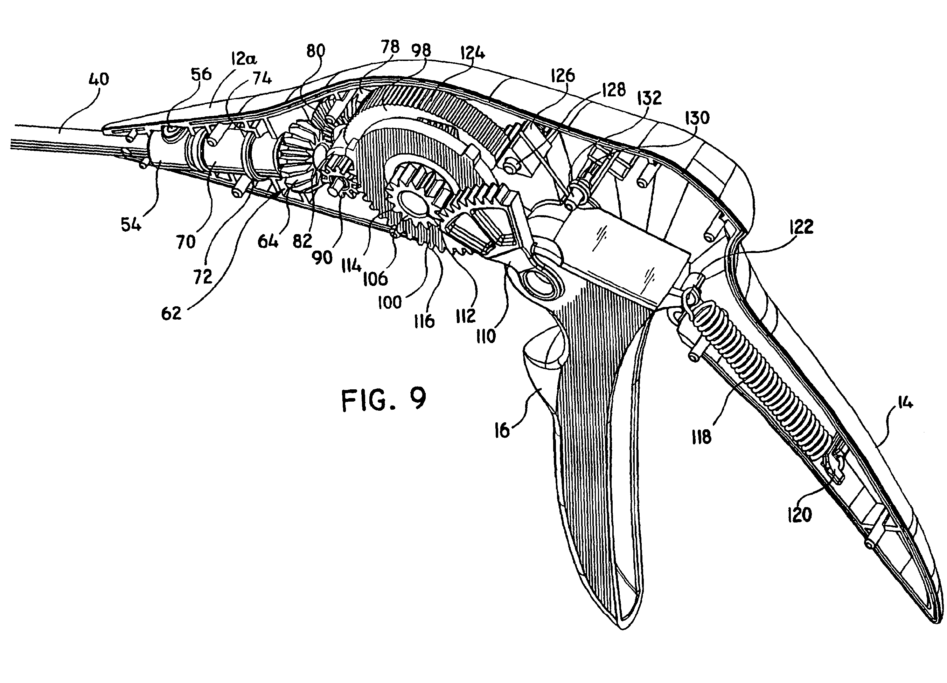

Referring now to FIGS. 8 and 9, coil fastener applier 10 is provided with a hollow bearing 54 having a port 56 formed in one side thereof. Port 56 is provided to receive proximal end 38 of drive rod 30 in order to rotate drive rod 30 as bearing 54 is rotated. Hollow bearing 54 additionally includes a keyed opening 58 formed in a proximal face thereof. A first bevelled gear 62 is provided to rotate bearing 54 and generally includes a plurality of teeth 64 and a shaft 66 extending distally from teeth 64. First bevelled gear 62 includes a keyed distal end 68 which is configured to securely engage the keyed opening 58 in hollow bearing 54. Thus, upon rotation of first bevelled gear 62 drive rod 30 is rotated. As best seen in FIG. 8, first beveled gear 62 is oriented perpendicular to, and rotates about, a longitudinal axis x of elongated tubular portion 18. A hollow sleeve 70 is provided having a flange 72 which engages slots 74 in housing halves 12a and 12b. Sleeve 70 rotatably supports first bevelled gear 62 within housing 12.

First bevelled gear 62 forms a part of a drive assembly 76 provided to rotate drive rod 30 in a single direction. Drive assembly 76 additionally includes a second bevelled gear 78 having a plurality of teeth 80 configured to engage teeth 64 of first bevelled gear 62. As shown, first bevelled gear 62 is oriented perpendicularly to second bevelled gear 78. Thus, second bevelled gear 78 rotates in a plane parallel to longitudinal axis x. Second bevelled gear 78 is rotatably supported within housing 12 by means of a hub 82. A shaft 84 is secured within a bore 88 of hub 82. Shaft 84 includes a keyed end 86. A drive gear 90 is provided to rotate the drive assembly 76 and includes a keyed opening 92 for engagement with keyed end 86 of shaft 84. Drive gear 90 includes a plurality of teeth 94.

Coil fastener applier 10 additionally includes an actuation assembly 96 which, in combination with drive assembly 76, convert longitudinal motion of trigger 16 into rotary motion of drive rod 30. Actuation assembly 96 generally includes a ratchet-plate gear 98 and an idler gear 100, which are rotatably supported on a stud 102 formed in housing half 12a. A compression spring 104 is provided between ratchet-plate gear 98 and idler gear 100 in a manner described in more detail hereinbelow. A pair of trigger gears 106 are affixed to ratchet-plate gear 98 on either side thereof. It should be noted that trigger gears 106 as well as ratchet-plate gear 98, idler gear 100 and drive gear 90 all rotate in planes parallel to that of second bevelled gear 62 and thus of longitudinal axis x of elongated tubular portion 18.

As noted hereinabove, trigger 16 is movably mounted on housing 12. Trigger 16 is pivotably mounted about a stud 108 formed in housing halves 12a and 12b. Trigger 16 is provided with a pair of spaced apart gear portions 110 each having a plurality of teeth 112 which cooperate to engage and rotate teeth 114 on trigger gears 106. Thus, by pivoting trigger 16 about stud 108, gear portions 110 rotate trigger gears 106 and thus ratchet-plate gear 98 and idler gear 100. As shown, idler gear 100 includes a plurality of teeth 116 on an edge thereof. Teeth 116 are configured for engagement with drive gear 90 such that upon actuation of trigger 16 idler gear 100 is rotated to cause rotation of drive gear 90 and thus of drive rod 30. A return spring 118 is provided to bias trigger 16 into an initial position spaced apart from handle 14. Return spring 118 is affixed at one end to a stud 120 on housing half 12a and is affixed at an opposite end to a stud 122 on trigger 16.

Actuation assembly 96 additionally includes a ratchet and pawl mechanism which prevents return of trigger 16 to an initial position until trigger 16 has been fully depressed. Ratchet teeth 124 are preferably formed along an edge of ratchet-plate gear 98. A pawl 126 is pivotally mounted about a stud 128 on housing half 12a and is engageable with ratchet teeth 124. Further, a biasing spring 130 is provided to bias pawl 126 into engagement with ratchet teeth 124. Biasing spring 130 is mounted about a stud 132 on housing half 12a and generally includes a first end 134 configured to engage pawl 126 and a second end 136 which is affixed within a slot 138 formed in housing 12a.

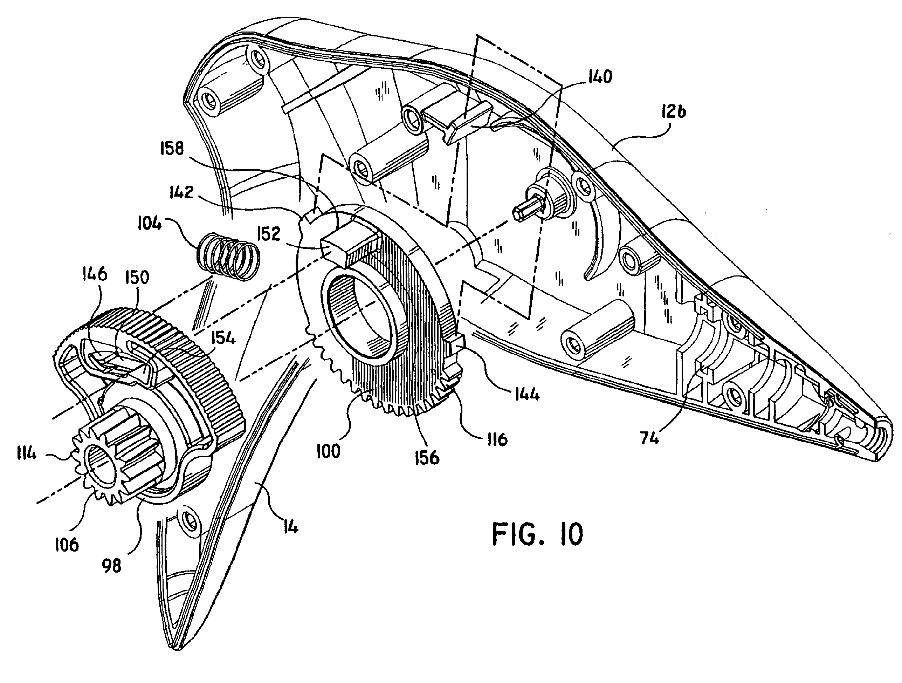

Referring now to FIG. 10, structure is provided to prevent more than one coil fastener 20 from being driven out of coil fastener applier 10 upon a single pull of trigger 16. Housing half 12b includes a blocking member 140 fixedly mounted to housing half 12b. Blocking member 140 is configured to engage first and second stops 142 and 144, respectively, formed on idler gear 100. Second stop 144 limits the degree of rotation of idler gear 100 during actuation of coil fastener applier 10 to install a coil fastener 20 and first stop 142 limits the rotation of idler gear 100 upon release of trigger 16 enabling coil fastener applier 20 to return to an initial position ready to install another coil fastener 20. By providing first and second stops 142 and 144 and blocking member 140, the operator can be assured that drive rod 30 will be rotated a predetermined number of times and that only a single coil fastener 20 will be driven from coil fastener applier 10 at a single time.

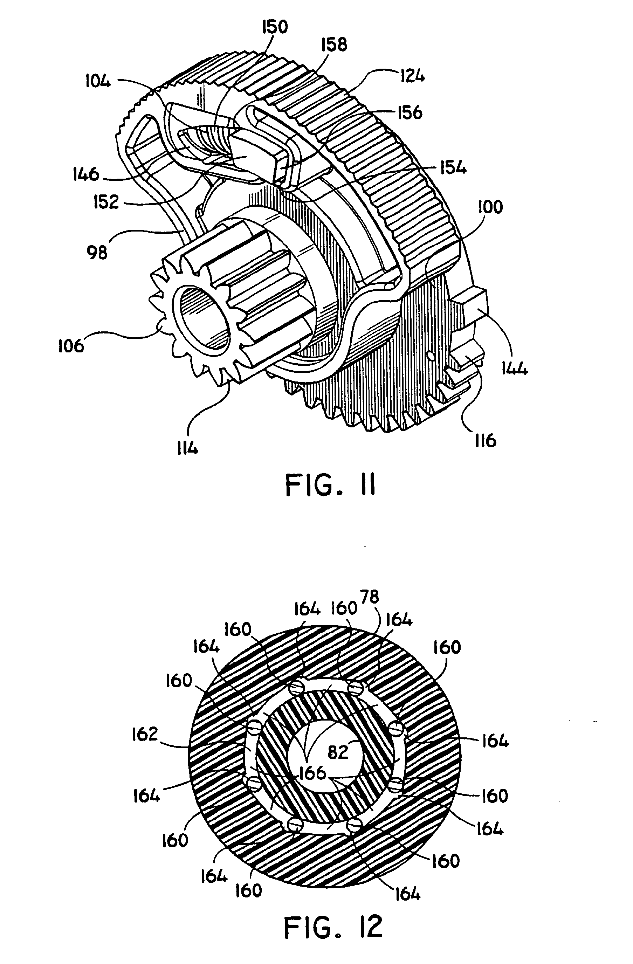

Turning now to FIGS. 10 and 11, ratchet-plate gear 98 is formed with a slot 146 which is configured to receive a compression spring 104 in a first portion 150 of slot 146. Compression spring 104 allows a slight amount of rotational movement to occur between ratchet-plate gear 98 and idler gear 100 during actuation and release of trigger 16 in a manner described in more detail hereinbelow. Idler gear 100 is formed with an engagement tab 152 projecting from a side thereof. Engagement tab 152 is positionable within a second portion 154 of slot 146. A first edge 156 of engagement tab 152 directly engages an edge 148 of ratchet-plate gear 98 while a second edge 158 of engagement tab 152 engages compression spring 104.

Referring now to FIG. 12, coil fastener applier 10 includes an anti-reverse mechanism which provides for a free return of hub 82 independent of second bevelled gear 78. This allows hub 82 to rotate second bevelled gear 78 in a driving or first direction when hub 82 is rotated in the first direction to thereby drive coil fastener 20 from coil fastener applier 10. The anti-reverse mechanism disengages hub 82 from second bevelled gear 78 when hub 82 is rotated in a second direction. This is desirable so as to prevent rotation of drive rod 30 in a direction opposite that of its driving direction which would rotate coil fastener 20 such that coil fastener 20 is withdrawn from tissue or mesh or is further withdrawn within tubular portion 18. The anti-reverse mechanism is a roller clutch which is formed between hub 82 and second bevelled gear 78. A plurality of roller pins 160 are provided in a circumferential space or gap 162 defined between hub 82 and second bevelled gear 78. Gap 162 includes enlarged release areas 164 and reduced grasping areas 166. Thus, as hub 82 is rotated in a first direction to move roller pins 160 into the grasping areas 166, roller pins 160 are cammed within grasping areas 166 to form a solid connection between hub 82 and second bevelled gear 78. Alternatively, when hub 82 is rotated in the opposite or second direction, it moves roller pins 160 into enlarged release areas 164 allowing hub 82 to rotate freely and independently of second bevelled gear 78.

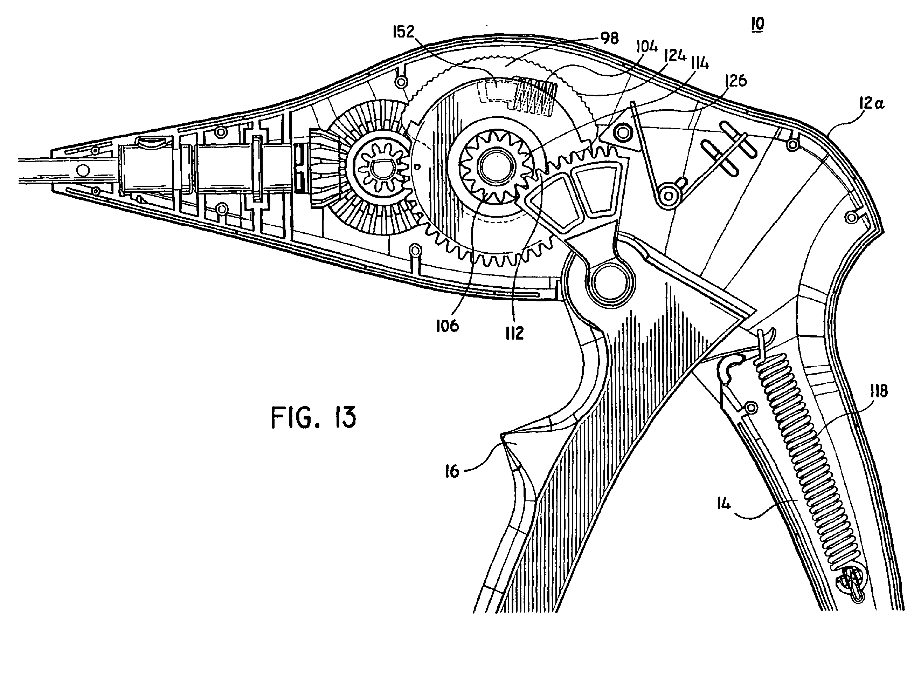

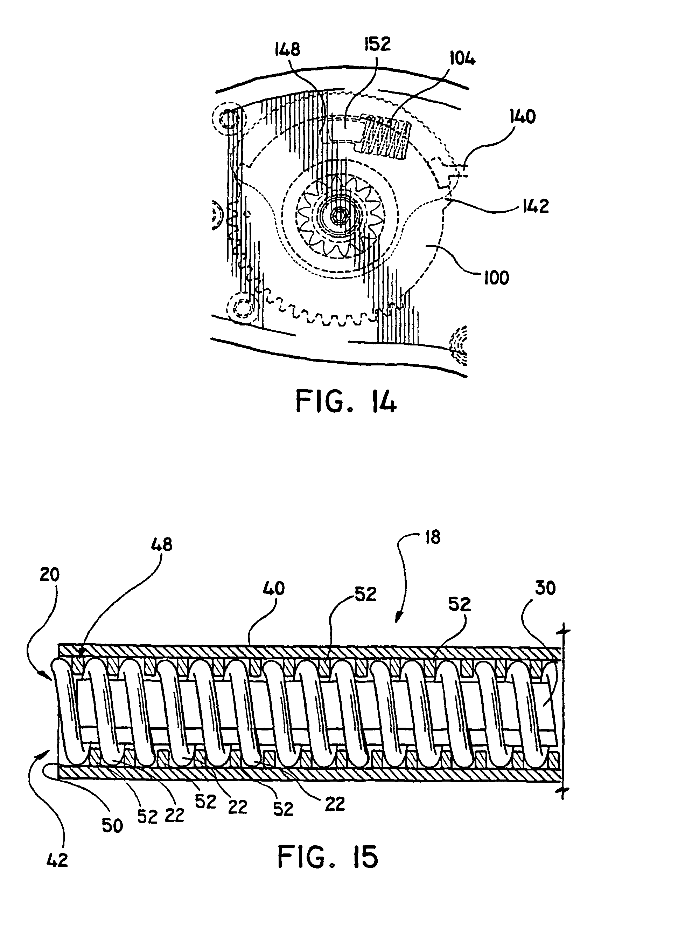

The operation of coil fastener applier 10 will now be described. Referring initially to FIG. 13, in an initial or starting position, trigger 16 is biased away from handle 14 due to the force of return spring 118. As shown, teeth 112 of trigger 16 are engaged with teeth 114 of trigger gears 106. Ratchet-plate gear 98 is in a counterclockwise most position, as viewed in FIG. 13, and pawl 126 is disengaged from teeth 124 of ratchet-plate gear 98. As best shown in FIG. 14, in the initial or starting position, blocking member 140 is engaged with first stop 142. In this position, with ratchet-plate gear in its counterclockwise most position and blocking member 140 engaged with first stop 142 of idler gear 100, engagement tab 152 is not engaged with edge 148 of ratchet-plate gear 98 but rather provides a slight compression to compression spring 104 as shown.

Referring to FIG. 15, within distal end 46 of tubular sleeve 40, a plurality of coil fasteners 20 are slidably mounted about drive rod 30 and positioned within tubular sleeve 40. Each coil body portion 22 of each coil fastener 20 engages surface 52 of coil spring 48 which, as noted above, is firmly secured to inner surface 50 of tubular sleeve 40.

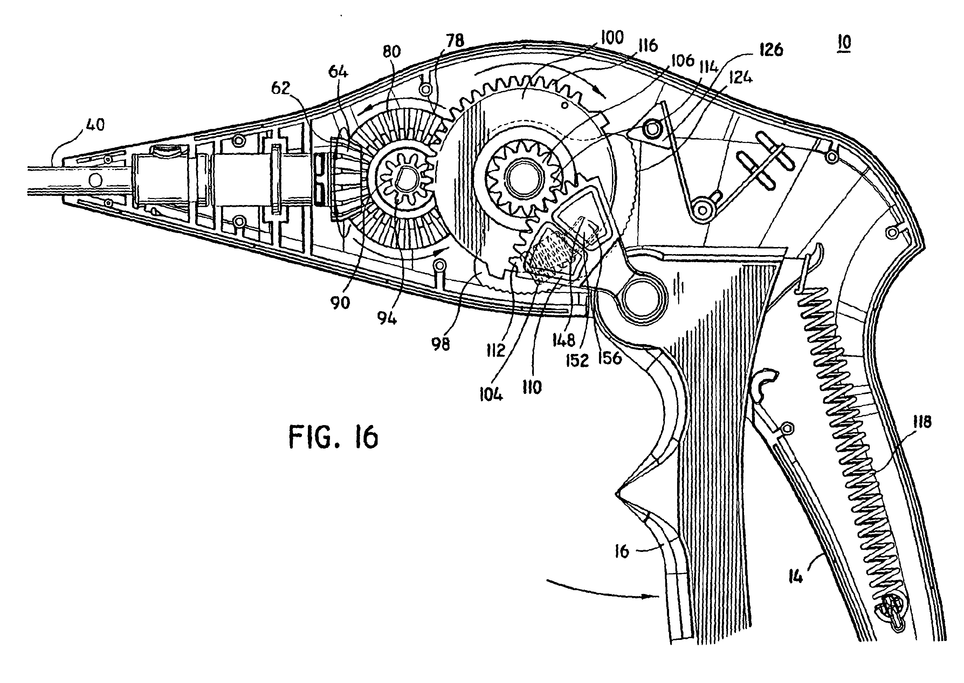

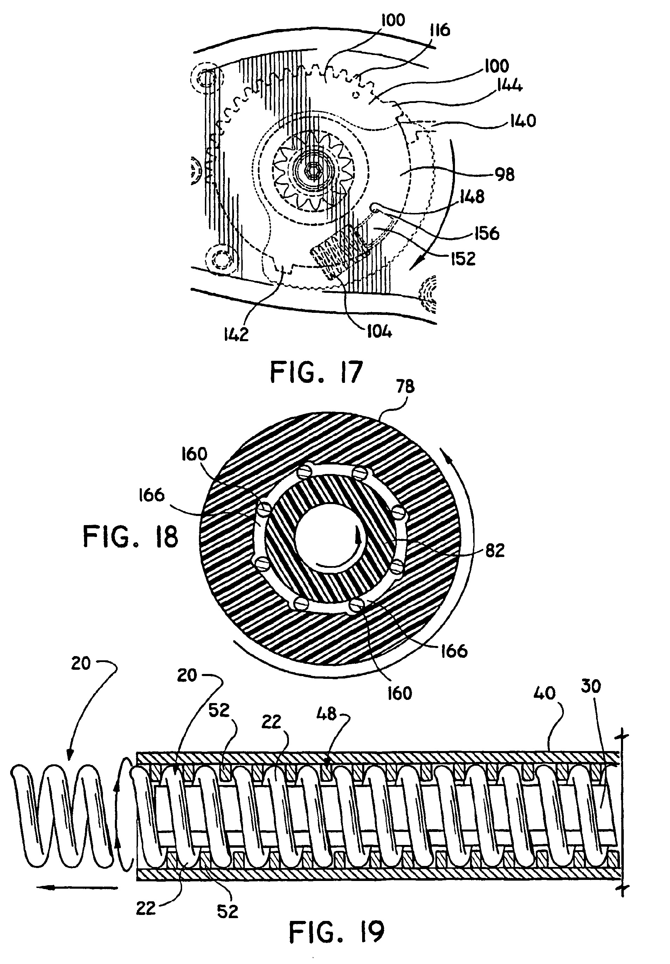

Referring now to FIG. 16, to actuate coil fastener applier 10, trigger 16 is drawn toward handle 14 against the bias of return spring 118. As trigger 16 is moved, teeth 112 on gear portions 110 of trigger 16 engage and rotate teeth 114 of trigger gears 106 clockwise as seen in FIG. 16. As shown in FIGS. 16 and 17, rotation of trigger gears 106, rotates ratchet-plate gear 98 such that first edge 156 of engagement tab 152 engages ratchet-plate gear 98. Idler gear 100 thus rotates with ratchet-plate gear 98 allowing a short expansion of compression spring 104. As idler gear 100 is rotated in a clockwise direction, as viewed in FIG. 16, teeth 116 of idler gear 100 engaged and rotate drive gear teeth 94 of drive gear 90 counterclockwise.

Referring now for the moment to FIG. 18, as drive gear 90 and thus hub 82, are rotated in a counterclockwise direction, the rotation of hub 82 causes roller pins 160 to be forced into the reduced grasping areas 166 of gap 162. Once moved into grasping areas 166, roller pins 160 form a solid and secure connection between hub 82 and second bevelled gear 78. Thus, second bevelled gear 78 is rotated in a counterclockwise direction as shown in FIGS. 16 and 18. Referring now again to FIG. 16, upon rotation of second bevelled gear 78 in a counterclockwise direction, teeth 80 of second bevelled gear 78 engage teeth 64 of first bevelled gear 62 to thereby rotate drive rod 30 within tubular sleeve 40.

Referring now to FIG. 19, as drive rod 30 is rotated within tubular sleeve 40, drive rod 30 rotates coil fasteners 20. Coil fasteners 20, being engaged with surface 52 of coil spring 48, are moved distally within tubular sleeve 40 by engagement of coil body portions 22 with surface 52. Thus, rotation of drive rod 30 rotates or screws a coil fastener out of the distal end of elongated tubular portion 18. As shown, this rotation of drive rod 30 also moves a next successive coil fastener 20 into position to be applied to tissue during a next cycling of coil fastener applier 10.

Referring back to FIG. 16, it should be noted that upon a complete depression of trigger 16, drive rod 30 is rotated precisely a predetermined amount such that only one coil fastener 20 is driven out of the distal end of elongated tubular portion 18, During compression of trigger 16, pawl 126 engages and rides over teeth 124 of ratchet-plate gear 98. Should handle 16 be stopped during depression at any intermediate position, pawl 126 is engaged with teeth 124 to ensure that ratchet-plate gear 98 and idler gear 100 are not rotated in an opposite direction thereby preventing only partial insertion or withdrawal of coil fastener 20, i.e. preventing a partial drive cycle. As shown in FIG. 16, upon complete depression of trigger 16, pawl 126 passes over teeth 124 and is disengaged therefrom.

Referring to FIG. 17, upon a complete depression of trigger 16, idler gear 100 rotates between a position wherein first stop 142 is rotated away from blocking member 140 until a position where blocking member 140 engages second stop 144 to thereby prevent further rotation of idler gear 100. This degree of rotation of idler gear 100 corresponds exactly to the amount of rotation of drive rod 30 necessary to completely drive a single coil fastener 20 out of elongated tubular portion 18 and into tissue.

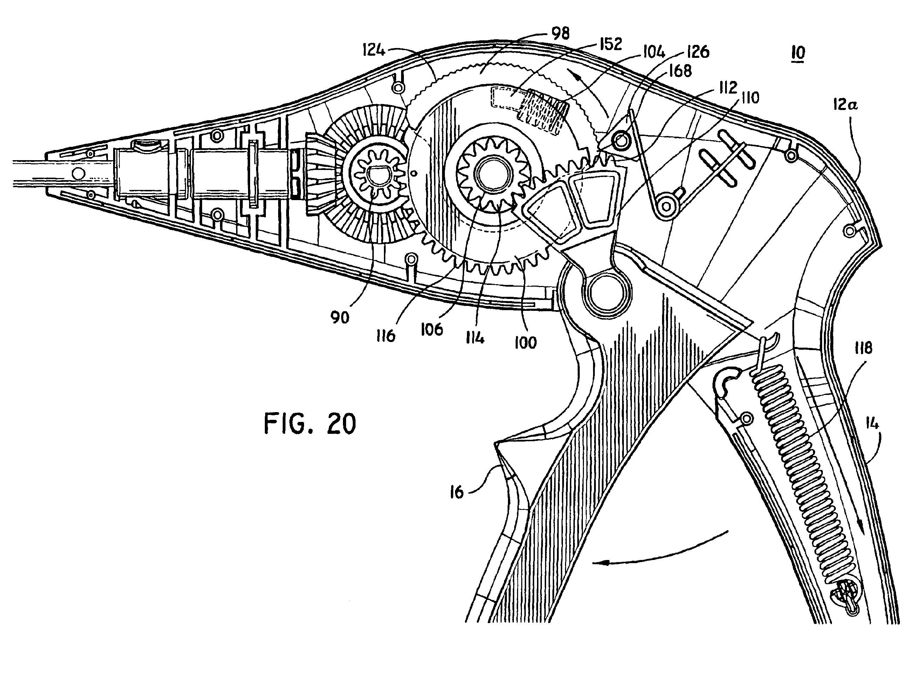

Referring now to FIG. 20, once trigger 16 had been completely depressed and a coil fastener 20 has been driven from elongated tubular portion 18 into tissue mesh or other suitable structure, trigger 16 may be released. Trigger 16 is then biased to an open or initial position due to the force of return spring 118. As trigger 16 is moved to an open position, teeth 112 of gear portions 110 rotate teeth 114 of trigger gears 106 counterclockwise, as viewed in FIG. 20, and thus ratchet-plate gear 98 in a counterclockwise direction. As ratchet-plate gear 98 is rotated in a counterclockwise direction, compression spring 104 forces idler gear 100 also in a counterclockwise direction. With idler gear 100 rotating in a counterclockwise direction, teeth 116 of idler gear 100 rotate drive gear 90 in a clockwise direction.

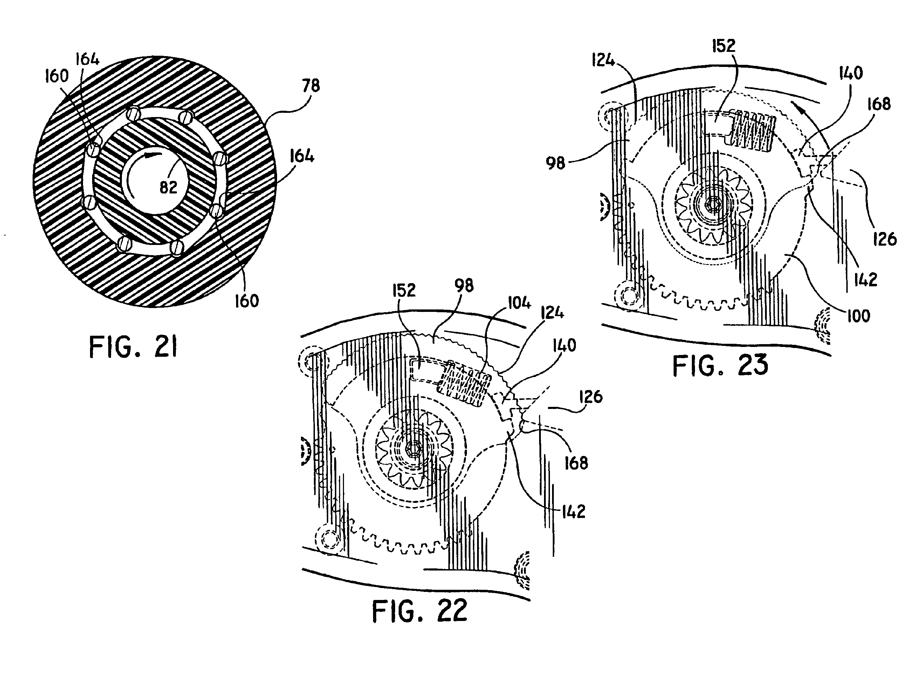

Referring now for the moment to FIG. 21, as noted hereinabove, coil fastener applier 10 includes an anti-reverse mechanism or roller clutch which disengages drive rod 30 from rotation upon release of trigger 16 and allows a free return of drive gear 90 to a start position. Thus, upon clockwise rotation of hub 82, hub 82 moves roller pins 160 into the enlarged release areas 164. Since clutch pins 160 no longer form a solid firm contact between hub 82 and second bevelled gear 78, hub 82 is free to rotate independently of second bevelled gear 78 thereby preventing any rotation of drive rod 30.

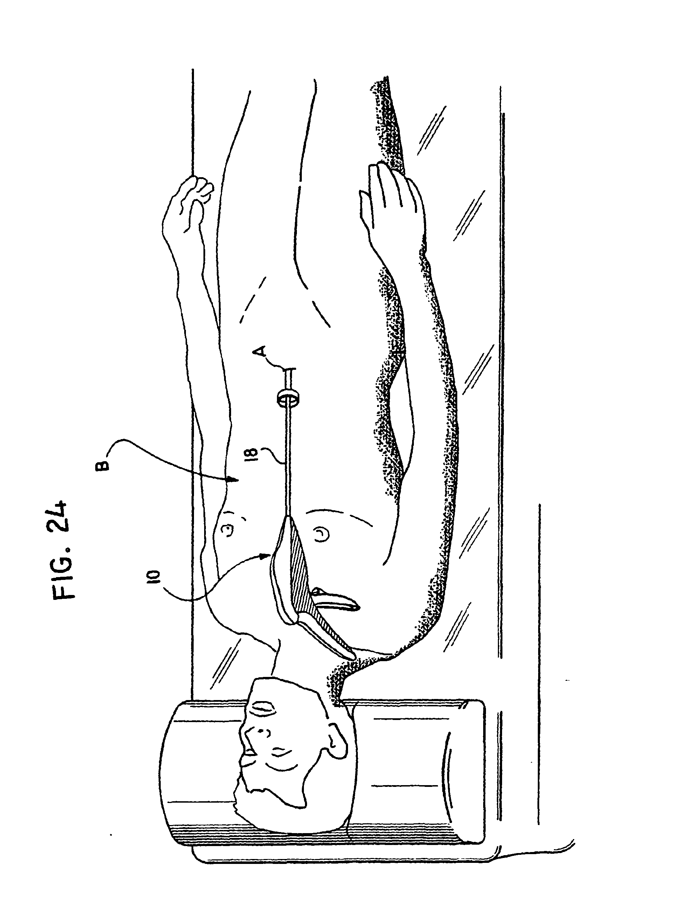

Referring to FIGS. 20 and 22 and 23, during release of trigger 16, pawl 126 moves along teeth 124 of ratchet-plate gear 98 until pawl 126 rests on a last tooth 168. This corresponds with the engagement of blocking member 140 with first stop 142 to thereby prevent any further rotation of idler gear 100. Once pawl 126 has reached its position on last tooth 168, the tension of return spring 118, being greater than the force of compression spring 104, forces trigger 16 a little further allowing trigger gears 106 to move ratchet-plate gear 98 slightly against the force of compression spring 104. As best shown in FIG. 23, the force of return spring 118 overcomes the force of compression spring 104 forcing engagement tab 152 to compress return spring 104. This compression of return spring 118 allows ratchet-plate gear 98 to move slightly enabling pawl 126 to move off of last tooth 168 of ratchet-plate gear 98. Thus, coil fastener apparatus 10 is returned to initial position ready to be actuated again and install another coil fastener.

Referring now to FIG. 24, coil fastener applier 10 is shown positioned through a small incision A made in a patient B for use in a surgical procedure, such as, for example, hernia repair.

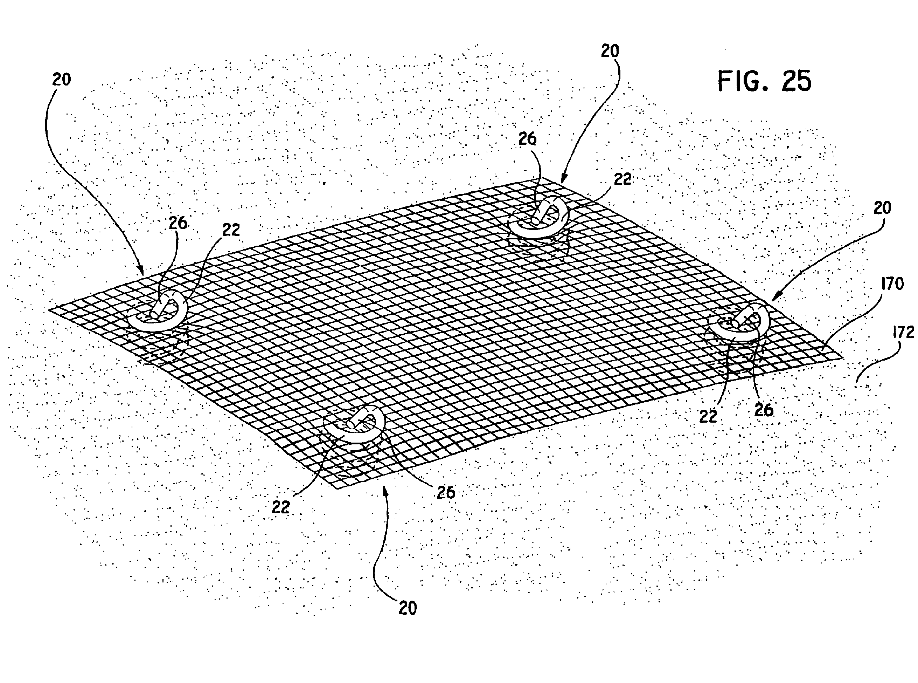

Referring now to FIG. 25, when used for hernia repair, surgical coil fastener applier 10 may be utilized to affix a portion of a suture mesh 170 to a tissue section 172. As shown in FIG. 25, several coil fasteners 20 may be utilized to secure mesh 170 to tissue 172. Preferably, in applying coil fasteners 20, coil fasteners 20 are rotated through mesh 170 and tissue 172 such that only approximately 180° of coil body portion 22 along with tang 26 extend externally of the mesh 170. Tang 26 provides an anchoring or securing mechanism to prevent mesh 170 from sliding off of coil body portion 22 of coil fastener 20.

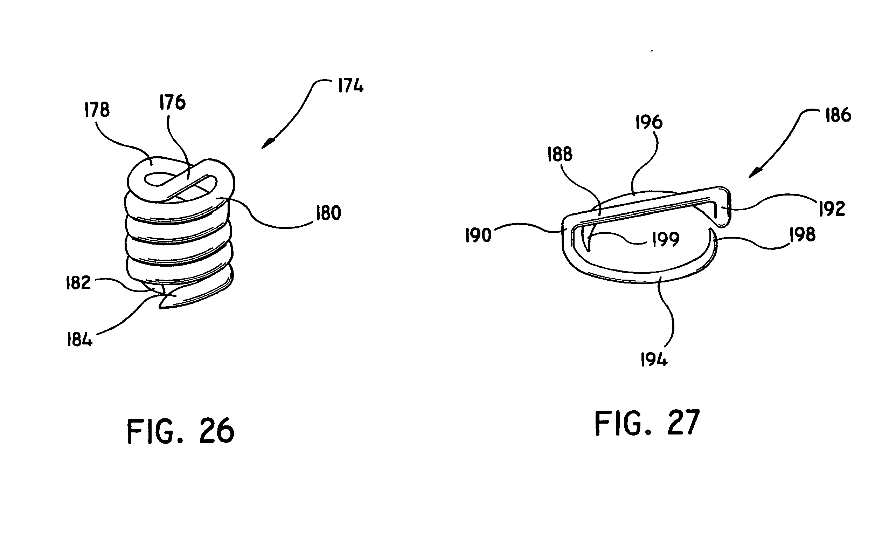

Referring now to FIGS. 26 and 27, there are disclosed alternate embodiments of coil fasteners suitable for use with coil fastener applier 10. Referring first to FIG. 26, an alternate embodiment coil fastener 174 is formed with a straight backspan 176 and helical coil body portions 178, 180 extending from each end of back span 176. Tissue penetrating points 182, 184 is provided at a free end of respective body portions 178, 180. Backspan 176 engages a slot extending completely through a drive rod (not shown) and is slidably supported thereon. Rotation of the drive rod rotates coil fastener 174 within sleeve 40 of coil fastener applier 10 and into tissue.

Referring now to FIG. 27, in a further alternative embodiment, a coil fastener 186 is formed with a straight backspan 188 having straight legs 190, 192 extending from each end of backspan 188 and which are parallel to each other. Semi-circular tissue penetrating portions 194, 196 terminating in tissue penetrating points 198, 199 extend from a free end of respective leg 190, 192. Semi-circular tissue penetrating portions 194, 196 are located in a common plane which is generally parallel to backspan 188. The backspan of the coil fastener also engages a completely slotted drive rod (not shown) and is rotated thereby.

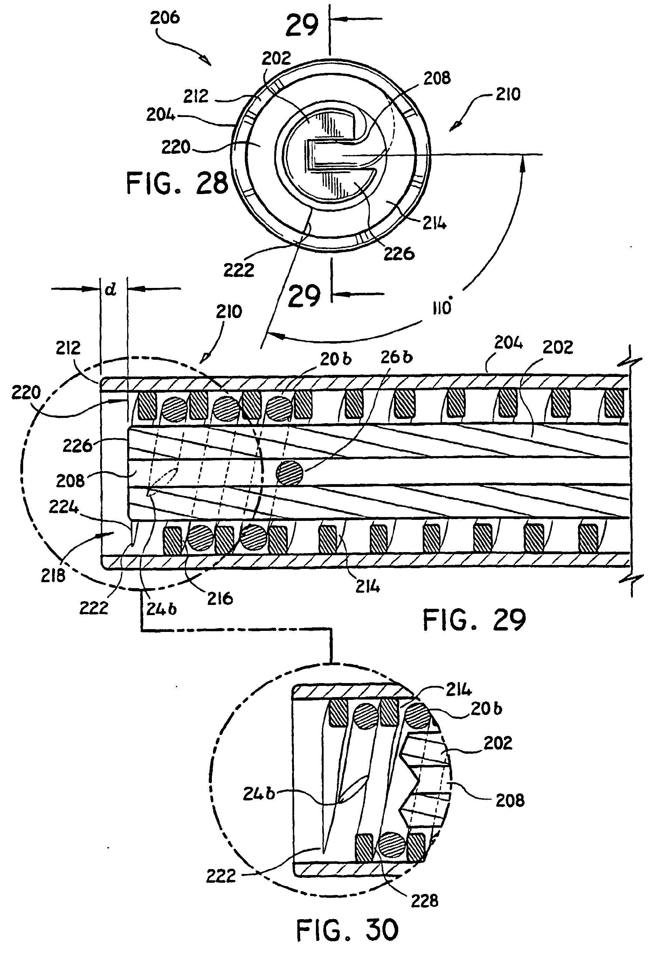

Referring now to FIGS. 28-37, there is disclosed an alternate embodiment of a coil fastener applier. Coil fastener applier 200, as described herein is substantially structurally and functionally identical to that described hereinabove including all the components included therein and can be used in exactly the same fashion to apply coil fasteners to tissue. Coil fastener applier 200 however includes additional structure, in the form of a locking mechanism, such that a drive rod 202 may be immobilized with respect to a tubular sleeve 204 thereby allowing coil fastener applier 200 to engage and remove a coil fastener, such as coil fastener 20, from tissue. Moreover, the design and dimensions of coil fastener applier 200 are such that elongated tubular portion 206 may be positioned over a coil fastener 20 which has been applied to tissue and engage a tang 26 of coil fastener 20 with a slot 208 in drive rod 202. The entire coil fastener applier 200 may then be rotated to remove coil fastener 20 from tissue.

Referring to FIGS. 28-30, a distal end 210 of tubular sleeve 204 terminates in a distalmost edge 212. As with coil fastener applier 10 above, a coil spring 214 is braised or welded to form a helical surface 216 within bore 218 of tubular sleeve 204. Coil spring 214 includes a distal end 220 which terminates in a distal tip 222. Distal end 220 has a flat ground surface 224 which sections or reduces the diameter of tip 222. Drive rod 202 terminates in a distal face 226. As shown in FIG. 28, slot 208 forms an angle of approximately 110° with distal tip 222 of coil spring 214. This angle allows the coil fastener to rotate three full turns in order to be released from coiled spring 214.

As illustrated in FIG. 29, in order to enable a tang 26 of a coil fastener 20 to enter slot 208, the distance "d" between distalmost edge 212 of tubular sleeve 204 and distal face 226 of drive rod 202 must be at least the wire diameter of a surgical fastener 20.

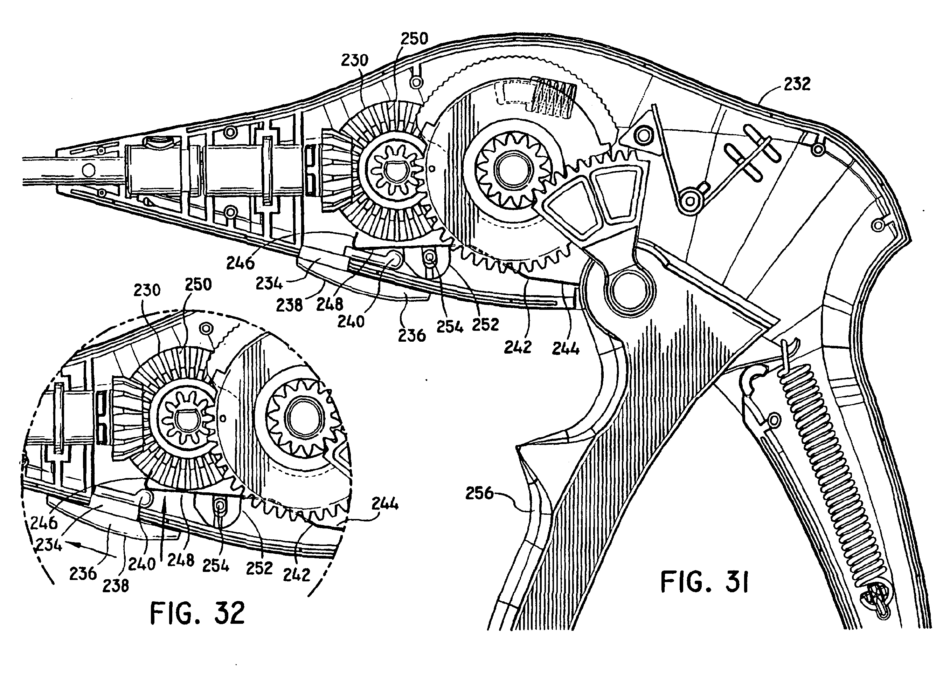

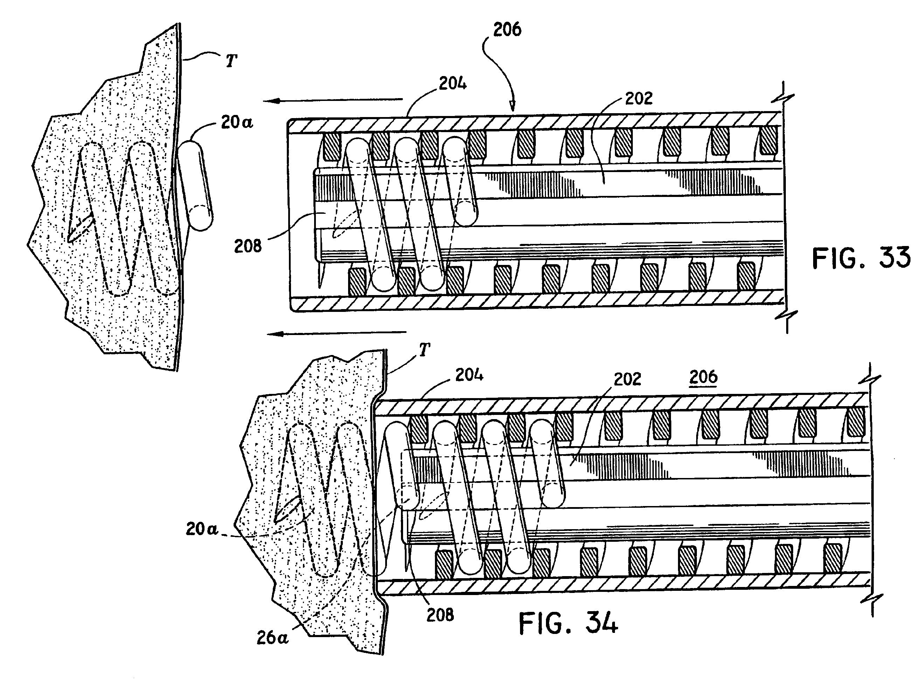

Preferably, this distance "d" is approximately 0.76mm (0.030 inches) and at least approximately 0.64 mm (0.025 inches) depending on the particular coil fastener being used. The distance between distal face 226 of drive rod 202 and distalmost edge 212 of tubular sleeve 204 in combination with the 110° angle between distal tip 222 and slot 208 of drive rod 202 (FIG. 28) enables slot 208 and drive rod 202 to engage at least half of the wire diameter of a tang 26 as elongated tubular portion 206 is positioned about a coil fastener 20 (FIG. 33) previously applied in tissue. FIGS. 29 and 30 illustrate a second coil fastener 20b positioned within elongated tubular portion 206 such that a tang 26b engages slot 208. A point 24b of second coil fastener 206 is in an initial position prior to actuation of coil fastener applier 200 and remote from distal face 226 of drive rod 202.

As noted above, in order to utilize coil fastener applier 200 to remove a coil fastener 20 from tissue, it is preferable to immobilize drive rod 202 relative to tubular sleeve 204. This can be accomplished by blocking the drive assembly, and, in particular, second bevelled gear 230, against movement thereby preventing rotation of drive rod 202 (FIG. 31).

Referring now to FIGS. 31 and 32, coil fastener applier 200 may be provided with a housing 232 containing a slot 234 in which a lockout button 236 is slidably mounted. Lockout button 236 is provided to immobilize drive rod 202 and, additionally, may serve as a shipping interlock to prevent actuation of coil fastener applier 200 prior to desired use. Lockout button 236 includes a finger surface 238 projecting outwardly away from housing 232. Lockout button 236 also includes cam 240 projecting into housing 232.

A blocking member or leaf spring 242 is provided and is affixed at its proximal end 244 to housing 232. A hook 246 may be provided at a distal end 248 of leaf spring 242 and is configured to engage teeth 250 of second bevelled gear 230. When hook 246 engages teeth 250, second bevelled gear 230, and thus drive rod 202, is prevented from rotation. In order to move hook 246 into engagement with teeth 250, a pivot 252 is rotatably mounted about a pivot pin 254 provided on housing 232. As shown in FIG. 31, in a proximalimost position, lockout button 236 and thus cam 240 does not engage leaf spring 242. Referring to FIG. 32, as lockout button 236 is moved in a distal direction, cam 240 drives hook 246 on distal end 248 into engagement with teeth 250 on second bevelled gear 230 thereby immobilizing drive rod 202. Additionally, hook 246 may be moved into engagement with teeth 250 by a complete firing of trigger 256 as described hereinbelow.

Referring now to FIGS. 32-36, use of coil fastener applier 200 to remove a coil fastener 20 which had previously been imbedded in tissue will now be described. Initially, lockout button 236 is moved distally to engage hook 246 with teeth 250 thereby immobilizing drive rod 202 (FIG. 32). Elongated tubular portion 206 is advanced toward a coil fastener 20a previously positioned in a tissue T and tubular sleeve 204 is positioned about coil fastener 20a such that a tang 26a of coil fastener 20a is positioned within slot 208 in drive rod 202. (See FIGS. 33 and 34).

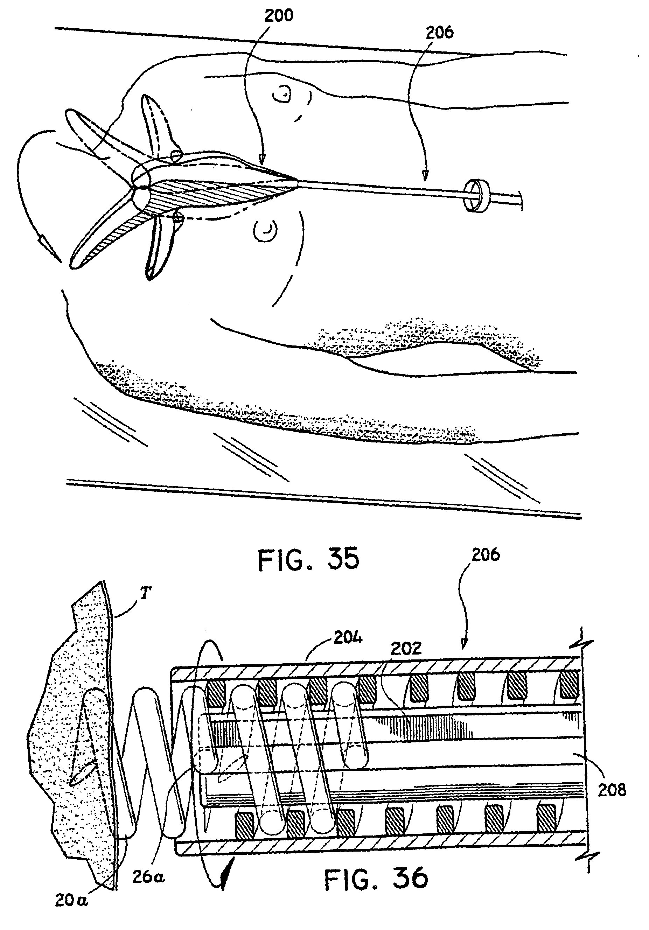

Referring now to FIGS. 35 and 36, the entire coil fastener applier 200 may be rotated to drive coil fastener 20 further into tissue T or to withdraw coil fastener 20 from tissue T. Coil fastener applier 200 is rotated to rotate elongated tubular portion 206 and thus drive rod 202, simultaneously with tubular sleeve 204, to withdraw coil fastener 20a from tissue T. In this manner, coil fastener applier 200 is uniquely configured and dimensioned to re-engage a tang 26a of coil fastener 20a previously applied to tissue and rotate coil fastener 20a out of tissue.

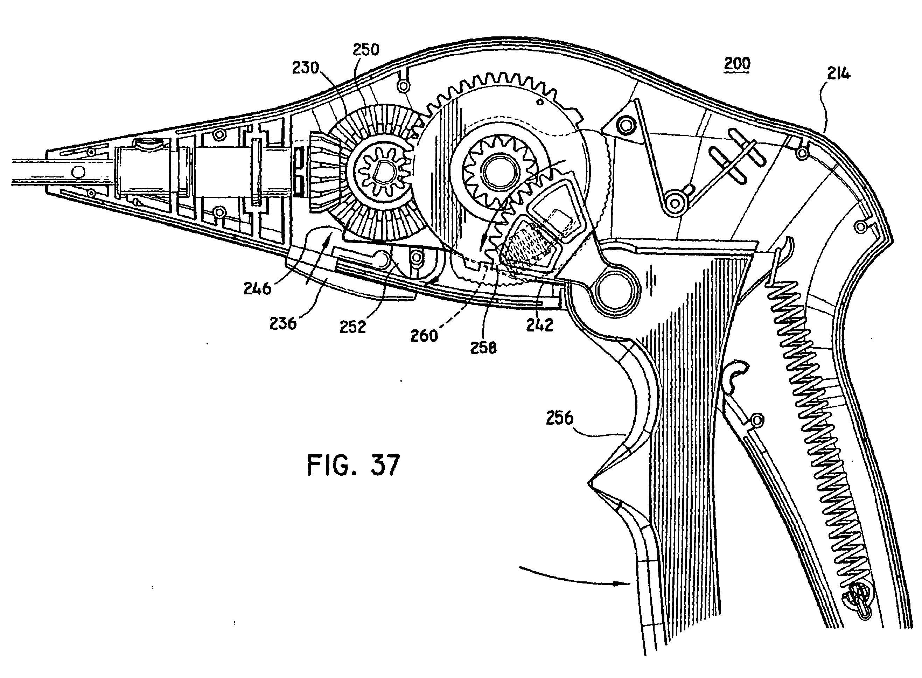

FIG. 37 illustrates an alternative method of engaging hook 246 with teeth 250 on second bevelled gear 230 is shown. Specifically, lockout button 236 is in a proximalmost or free to fire position. Once trigger 256 has been pulled to a full firing stroke, idler gear 258 engages a proximal portion 260 of leaf spring 242 thereby pivoting leaf spring 242 about pivot 252. As leaf spring 242 is pivoted about pivot 252, hook 246 is driven into engagement with teeth 250 on second bevelled gear 230. Thus, at the end of any individual firing stroke, drive rod 202 is immobilized with respect to tubular sleeve 204 and the entire instrument 200 may be rotated to either further advance or retract a coil fastener 20 with respect to tissue. Alternatively, trigger 256 may be released to an initial position to begin a second firing stroke.

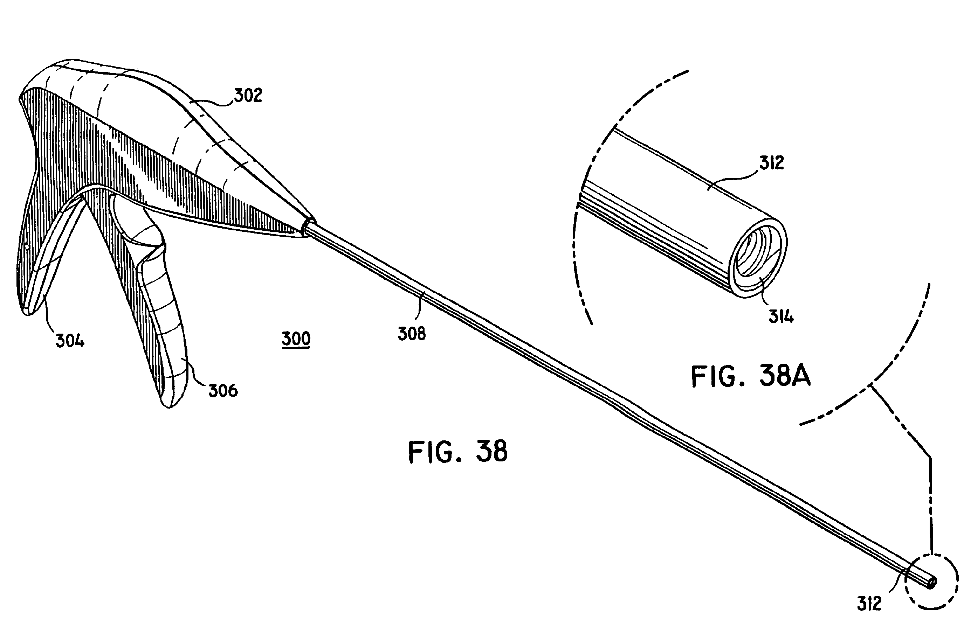

Referring now to FIGS. 38 to 57, there is disclosed an alternate embodiment of a coil fastener applier. Coil fastener applier 300, as described herein, is substantially structurally and functionally identical to the embodiments described hereinabove. Any structural or functional variations therefrom are described in detail hereinbelow.

Referring initially to FIG. 38, coil fastener applier 300 generally includes a housing 302 which may be formed as separate halves and a handle portion 304 extending from housing 302. A trigger 306 is movably mounted to housing 302. An elongated tubular portion 308 extends distally from housing 302.

Referring to FIG. 38A, elongated tubular portion 308 includes threads 314 therein to facilitate rotating coil fasteners out of elongated tubular portion 308.

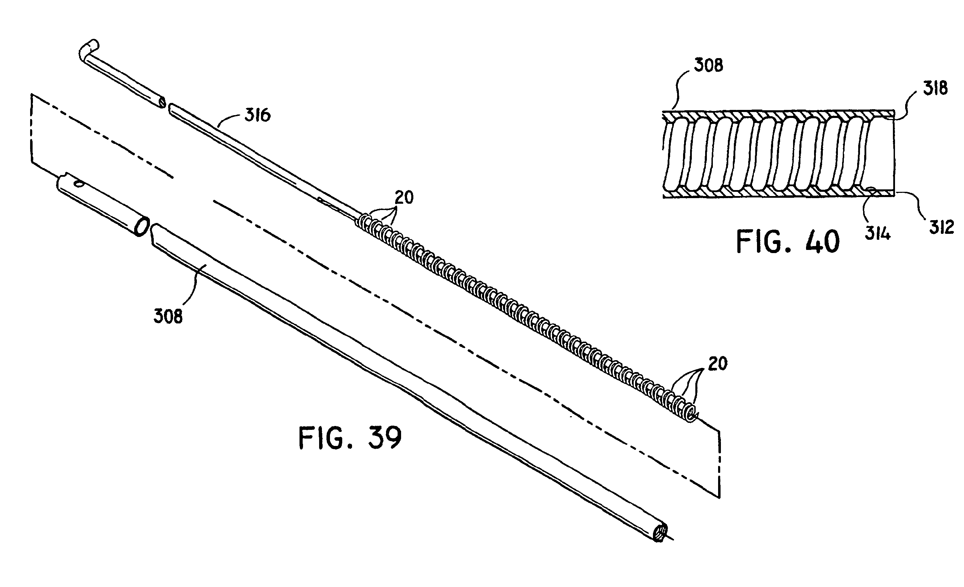

Referring now to FIG. 39, elongated tubular portion 308 includes a drive rod 316 rotatably mounted therein. Drive rod 316 supports a plurality of coil fasteners, such as, for example, coil fasteners 20 described hereinabove. As shown in FIG. 40, and in contrast to previous embodiments, threads 314 are integrally formed in an inner surface 318 of elongated tubular portion 308. Threads 314 may be formed within elongated tubular portion 308 by known machining or die stamping methods and are preferably formed by roll-forming threads 314 into elongated tubular portion 308. Threads 314 do not extend completely to distal end 312 of elongated tubular portion 308.

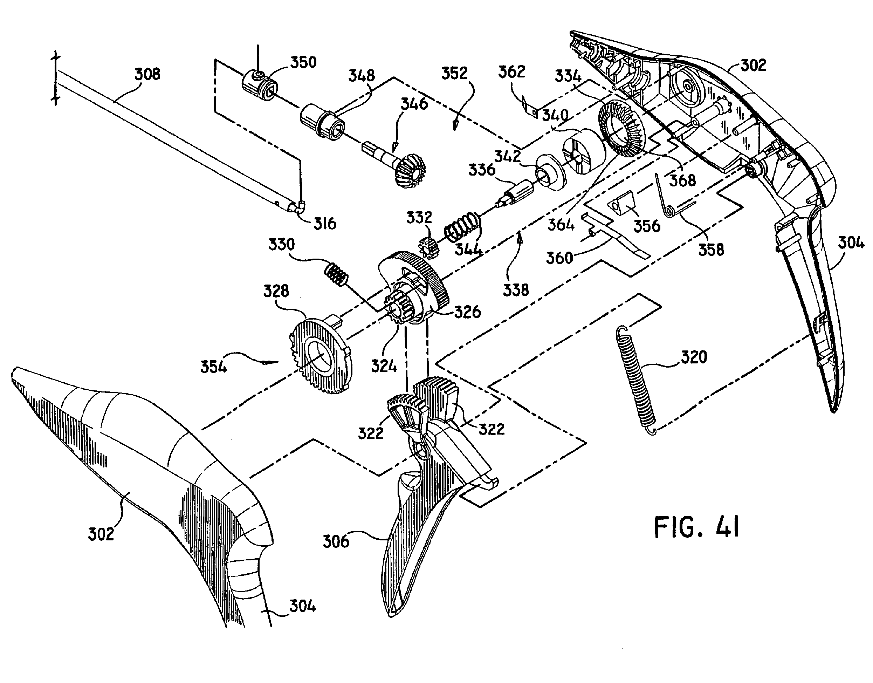

Referring now to FIG. 41, as noted hereinabove, coil fastener applier 300 is substantially structurally identical to previous embodiments. Thus, coil fastener applier 300 is provided with trigger 306 pivotally mounted to housing 302. A spring 320 is provided between trigger 306 and handle 304. Trigger 306 includes gear portions 322 which are configured to engage and rotate a trigger gear 324 mounted to a ratchet plate gear 326. An idler gear 328 is also provided. A spring 330 is positioned between ratchet plate gear 326 and idler gear 328. Ratchet plate gear 326 differs from ratchet plate gears above in a manner described in more detail below. Idler gear 328 is engageable with a drive gear 332 to rotate a second bevelled gear 334. Drive gear 332 is mounted on a shaft 336. In a variation from previous embodiments, the roller-type clutch is replaced with a ratchet plate clutch 338. Ratchet plate clutch 338, including a first ratchet plate 340, a second ratchet plate 342 and a spring 344, is provided to engage and disengage drive gear 332 from second bevelled gear 334 as described hereinbelow.

Second bevelled gear 334 drives a first beveled gear 346. Rotation of first bevelled gear 346 rotates a hollow sleeve 348 and a hollow bearing 350 and thus drive rod 316 in a manner substantially identical to that described hereinabove with respect to previous embodiments. As with previous embodiments first and second bevelled gears, 340 and 334, form part of a drive assembly 352 while idler gear 328 and ratchet plate gear 326 form part of an actuation assembly 354.

Housing 302 additionally includes a pawl 356 engagable with ratchet plate gear 326. Pawl 356 is biased toward ratchet plate gear 326 by a spring 358. A leaf spring 360 may optionally be provided to engage second bevelled gear 334.

Notably, coil fastener applier 300 includes an anti-reverse mechanism for second bevelled gear 334 which differs from that provided on previous embodiments but which does not fall under the scope of claim 1. A blocking spring 362 is mounted to housing 302 and is configured to engage teeth 364 on second bevelled gear 334. Blocking spring 362 allows rotation of second bevelled gear 334 in a first direction to drive coil fasteners from elongated tubular portion 308 while blocking second bevelled gear 334 from rotating in a second and opposite direction.

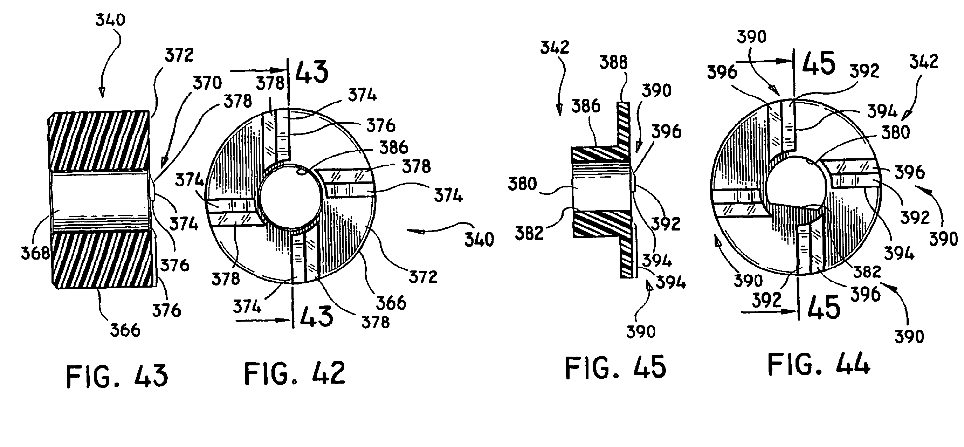

Turning now to FIGS. 42-47, the details of ratchet plate clutch 338 including first and second ratchet plates 340 and 342, respectively, will now be described. Referring to FIGS. 42 and 43 first ratchet plate 340 is formed as a cylinder having an outer surface 366 which is dimensioned and configured to firmly seat within a bore 368 of second bevelled gear 334 (FIG. 41). First ratchet plate 340 defines a bore 370 within which shaft 336 is received. First ratchet plate 340 freely rotates about shaft 336. In order to engage second ratchet plate 342 and thereby alternately fixedly and releasably engage second bevelled gear 334 with shaft 336, first ratchet plate 340 is provided with four equally spaced ratchet teeth 370 on an end face 372 thereof. Ratchet teeth 370 are preferably spaced 90* apart and extend across end face 372 from bore 386 radially outwardly toward outer surface 366. Each of ratchet teeth 370 includes a block portion 374 having an engagement face 376 and an angled surface 378 which slopes from block portion 374 to end face 372. Ratchet teeth 370 cooperate with corresponding teeth on second ratchet plate 342 to alternately fixedly and releasably engage first ratchet plate 340 with second ratchet plate 342.

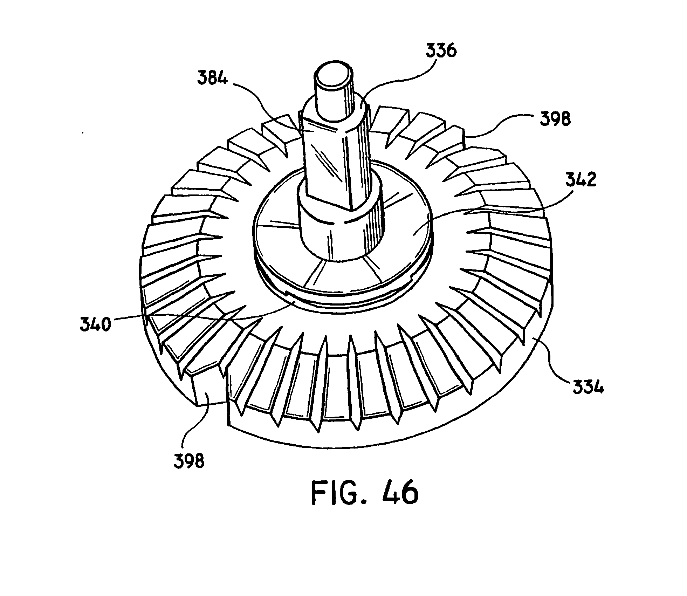

Referring now to FIGS. 44 and 45, second ratchet plate 342 defines a bore 380 which is configured to fixedly receive shaft 336. Specifically, bore 380 includes a keyed or flat surface 382 which engages a corresponding keyed or flat surface 384 on shaft 336 (FIG. 46). Second ratchet plate 342 includes a cylinder 386 and a larger diameter portion 388. Larger diameter portion 388 provides an abutment surface against which spring 344 can bias second ratchet plate 342 toward first ratchet plate 340. Ratchet teeth 390 are provided on an end face 392 of larger diameter portion 388. Ratchet teeth 390 are similar and complimentary to ratchet teeth 370 on first end plate 340. Specifically, each of ratchet teeth 390 includes a block portion 392 having an engagement face 394 and an angled surface 396.

FIG. 46 illustrates first ratchet plate 340 positioned within second bevelled gear 334 and shaft 336 positioned within second ratchet plate 342. Second bevelled gear 334 includes two notches 398 for receipt of blocking structure formed on ratchet plate gear 326 as described hereinbelow.

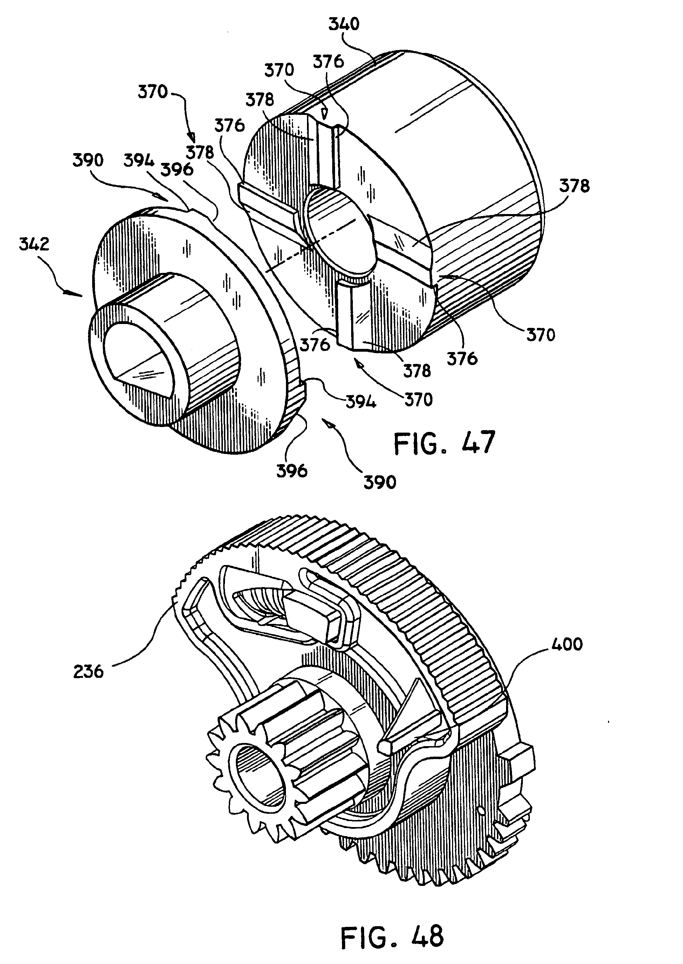

Referring to FIG. 47, ratchet teeth 370 on first ratchet plate 340 are configured to fixedly engage ratchet teeth 390 on second bevelled gear 342 when second bevelled gear 342 is rotated in a first direction corresponding to actuation of coil fastener applier 300. Ratchet teeth 370 sidlingly or releasably engage ratchet teeth 390 when second ratchet plate 342 is rotated in a second and opposite direction corresponding to release and return of trigger 306 after firing coil fastener applier 300. Specifically, engagement faces 376 fixedly engage engagement faces 394 when second ratchet plate 342 is rotated in the first direction and angled surface 378 slide over or releasably engage angled surfaces 396 when second ratchet plate 342 is rotated in the second direction. While constrained to rotate with shaft 336, second ratchet plate 342 is free to slide along the length of shaft 336 against the bias of spring 344 when rotated in the second direction.

As noted above, blocking structure is provided on ratchet plate gear 326 to engage notches 396 on second bevelled gear 334. Referring now to FIG. 48, ratchet plate gear 326 includes a stud 400 which engages one of notches 396 when coil fastener applier 300 is in an unfired condition.

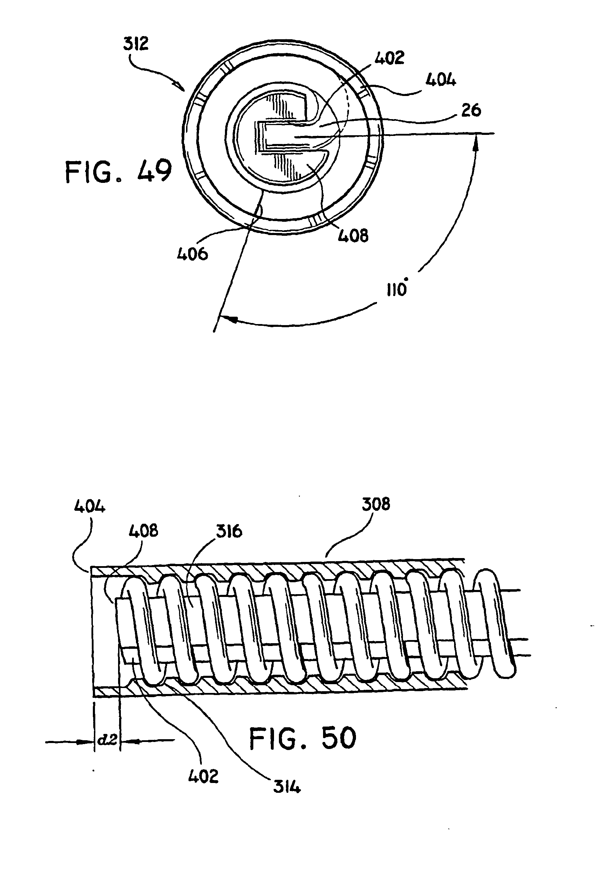

As with the previous embodiment, coil fastener applier 300 is designed such that elongated tubular portion 308 may be positioned over a coil fastener 20 which has been applied to tissue and engage a tang 26 of coil fastener 20 with a slot 402 in drive rod 316 (FIG. 49). The entire coil fastener applier 300 may then be rotated to remove coil fastener applier 20 from tissue. Additionally, coil fastener applier 300 is designed to engage tang 26 of coil fastener 20 previously applied to tissue and drive coil fastener 20 further into the tissue.

Referring now to FIG. 49, distal end 312 of elongated tubular portion 308 terminates in a distalmost edge 404. Threads surface or threads 314 are roll formed into elongated tubular portion 308 and terminate in a distal tip 406. Drive rod 316 terminates in a distal face 408. As shown, slot 402 forms an angle β with distal tip 406 of thread 314. In order to ensure a sufficient grip on tang 26 of coil fastener 20 previously applied to tissue β is approximately 110°.

Referring to FIG. 50, in order to enable tang 26 of coil fastener 20 to enter sot 402 of drive rod 316, the distance d2 between distalmost edge 404 of elongated tubular portion 308 and distal face 408 of drive rod 316 is approximately 0.76 nm (0.030 inches) and at least 0.64 (0.025 inches) depending upon the wire diameter of coil fastener 20. As shown, threads 314 end proximally of distal face 408 of drive rod 316. As noted above, the specific combination of β and d2 enables slot 402 in dive rod 316 to engage tang 26 of a coil fastener 20 previously applied to tissue and withdraw or drive in further coil fastener 20 as coil fastener applier 300 is rotated.

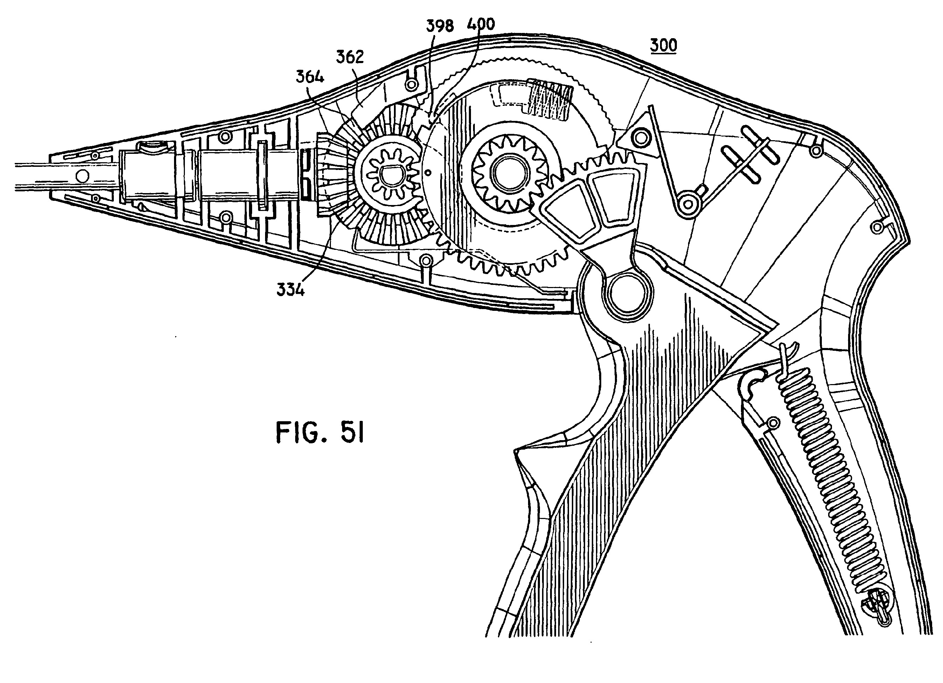

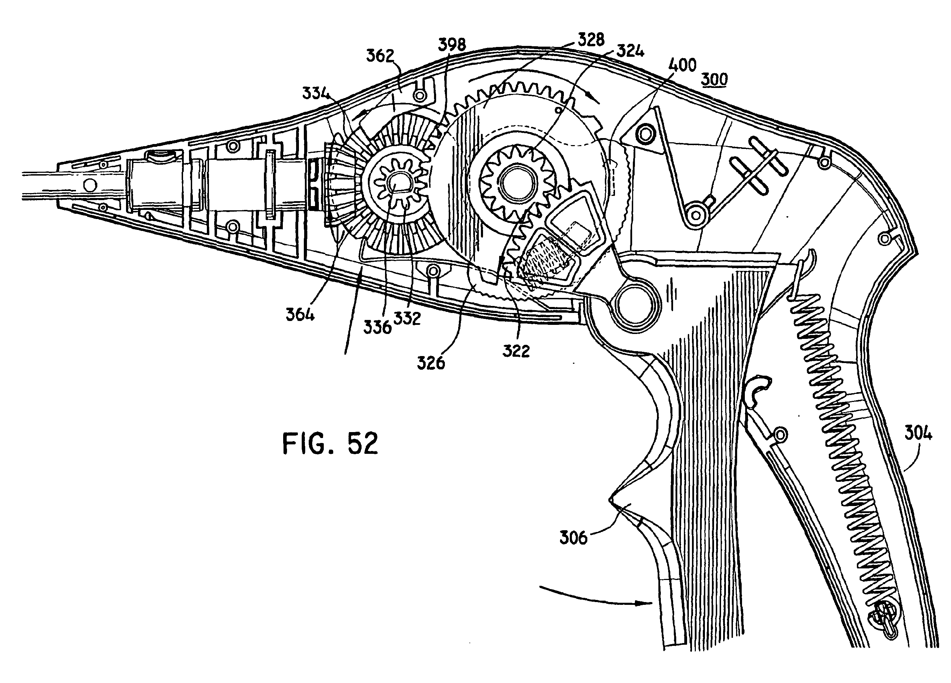

The operation of the coil fastener applier 300 to eject a coil fastener 20 therefrom will now be described. Referring to FIG. 51, in an initial and unfired position, stud 400 is positioned within notch 398 of second bevelled gear 334 thereby preventing inadvertent rotation of drive rod 316. Additionally, blocking spring 362 engages teeth 364 of second bevelled gear 334 thereby preventing rotation of second bevelled gear 334 in a second direction. Turning now to FIG. 52, as trigger 306 is depressed towards handle 304, trigger gear portion 322 drives trigger gear 324 and thus ratchet plate gear 326 in a manner similar to that described hereinabove. Stud 400 is rotated out of engagement of notch 398 in second bevelled gear 334. As second bevelled gear 334 rotates in the first direction, blocking spring 362 rides over teeth 364. Rotation of idler gear 328 rotates drive gear 332 similar to that described hereinabove and thus rotates shaft 336.

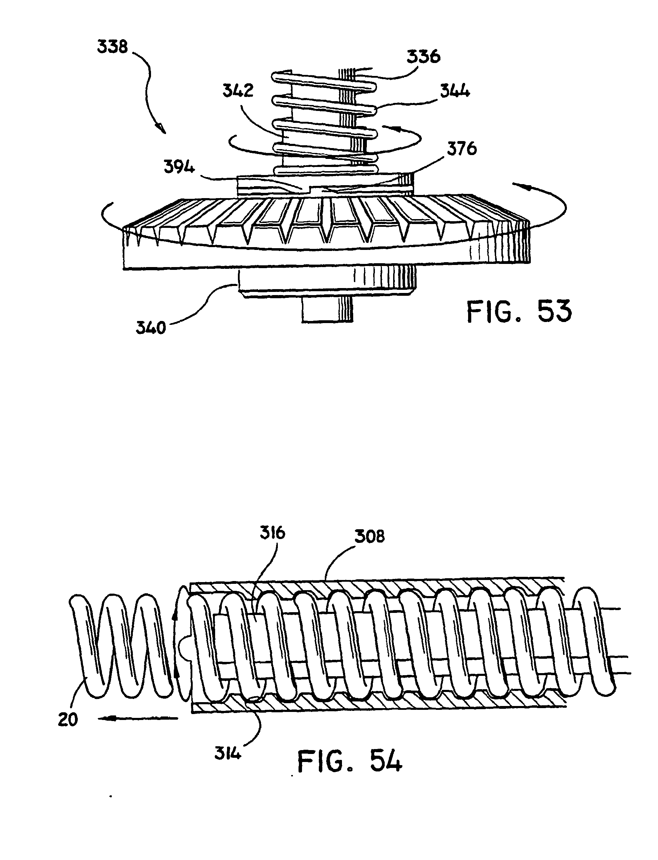

Referring now to FIG. 53, the operation of ratchet plate clutch 338 will now be described. Second ratchet plate 342, which is constrained to rotate with shaft 336, is biased against first ratchet plate 340 by spring 344. Thus, engagement face 394 of second ratchet plate 342 engages engagement face 376 of first ratchet plate 340 causing first ratchet plate 340 to rotate with second ratchet plate 342. First ratchet plate 340 rotates second bevelled gear 334 therewith. Rotation of second bevelled gear 334 in a first direction rotates drive rod 316 in a manner similar to that described hereinabove with respect to previous embodiments to eject a coil fastener 20.

Referring now to FIG. 54, as drive rod 316 is rotated, a coil fastener 20 is threaded out of distal end of elongated tubular portion 308 by engagement with threads 314. In this manner, coil fastener applier may be actuated to drive a coil fastener 20 into tissue and/or through hernia mesh and into tissue.

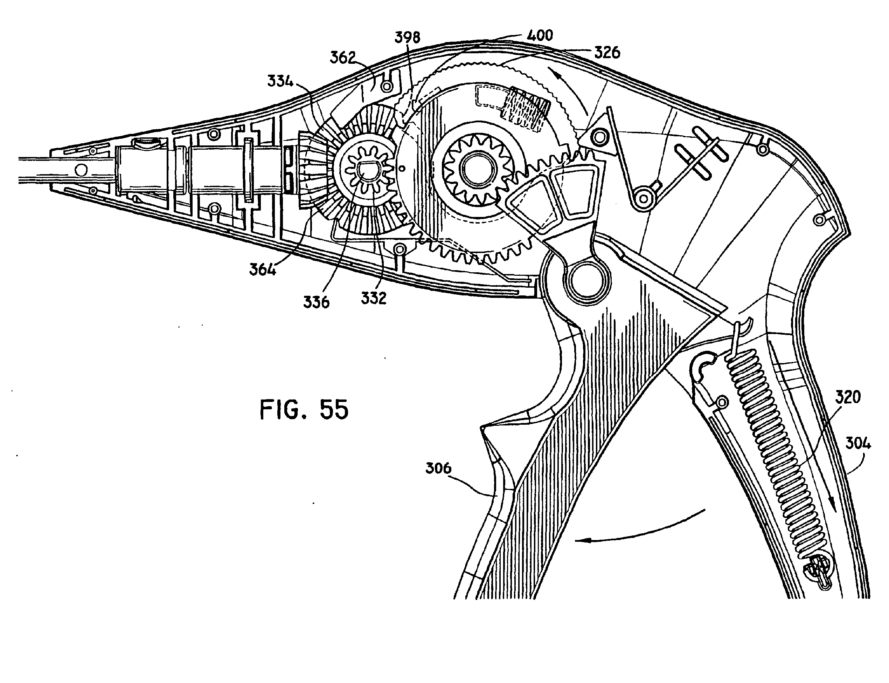

Referring now to FIG. 55, upon release of trigger 306, trigger 306 is biased away from handle 304 by means of spring 320. Release of trigger 306 causes ratchet plate gear 326 to rotate such that stud 400 advances towards notch 398 in second bevelled gear 334. Notably, second bevelled gear 334 is prevented from rotation by engagement of blocking spring 362 with teeth 364 on second bevelled gear 334. Rotation of idler gear 328 rotates drive gear 332 and thus, drive shaft 336.

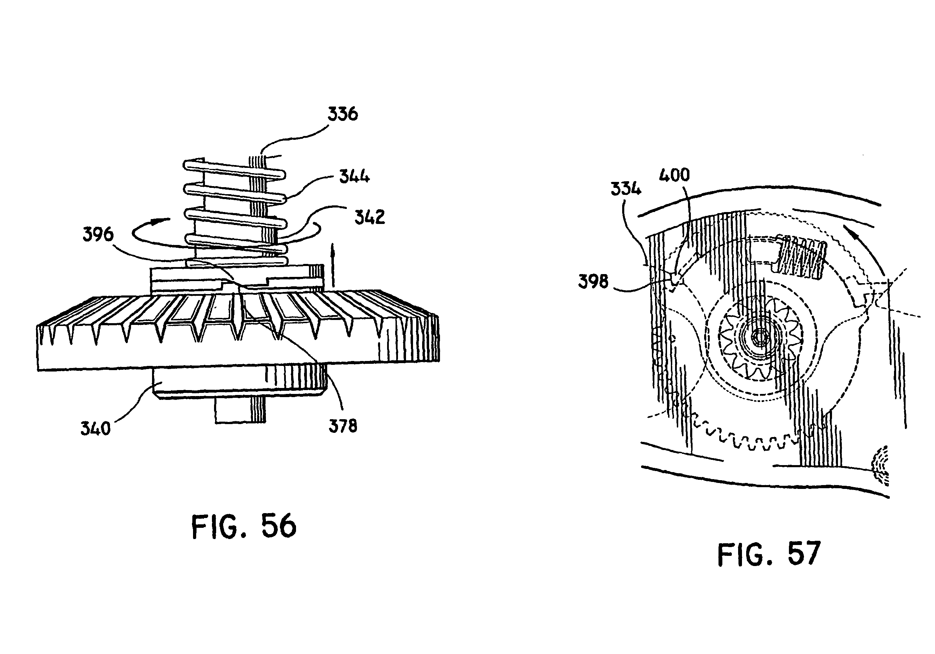

Referring now to FIG. 56, as drive shaft 336 is rotated in a second direction, it causes rotation of second ratchet plate 342 in a section direction. Rotation of ratchet plate 342 in a second direction causes engagement of angled surface 396 of second ratchet plate 342 with angled surface 378 of first ratchet plate 340. As second bevelled gear 334 is constrained from rotation by means of blocking spring 362 (FIG. 55), second ratchet plate 342 moves longitudinally with respect to shaft 336 and against the bias of spring 344.

Upon complete release of trigger 306, stud 400 re-engages notch 398 in second bevelled gear 334 to there again secure second bevelled gear 334 against rotation (FIG. 57).

As noted hereinabove, at this point, the entire coil fastener applier 300 may be rotated to further drive a coil fastener 20 deeper into tissue or may be utilized to re-engage a tang 26 of coil fastener 20 previously applied and remove coil fastener 20 from tissue. When coil fastener 20 are removed from tissue, an accessory instrument such as, for example, grasping instrumentation may be utilized to remove the unthreaded coil fastener from the surgical site.

It will be understood that various modifications may be made to the embodiments disclosed herein. For example, a shorter elongated tubular portion containing more or less coil fasteners may be provided for greater ease of handling during open surgery. Various articulations may be provided along the length of the elongated tubular portion to facilitate positioning of the coil fastener applier within the body. Additionally various configurations of the drive rod and slots or fastener retaining structure may be provided to accommodate various types of rotary fasteners. Further, alternate mechanisms may be provided to immobilize the drive rod relative to the elongated tubular portion or housing. Therefore, the above description should not be construed as limiting, but merely as exemplifications of preferred embodiments. Those skilled in the art will envision other modifications within the scope of the claims appended hereto.

## Claims

1. A coil fastener applier (10) comprising:
a housing (12) having a stationary handle (14) affixed to the housing;
an elongated tubular portion (18) extending distally from the housing;
a drive rod (30) rotatably mounted within the elongated tubular portion and having at least one coil fastener (20) releasably mounted on the drive rod;
a drive assembly (76) including at least one gear (62) engageable with the drive rod (30) for rotating the drive rod;
an actuation assembly (96) engageable with the drive assembly; and
a trigger (16) movably mounted on the housing and engageable with the actuation assembly (96), wherein movement of the trigger in a first direction rotates the drive rod (30) to drive a coil fastener (20) distally;
wherein the drive assembly (76) further includes an anti-reverse mechanism so as to prevent rotation of drive rod (30) in a direction opposite that of its driving direction, **characterized in that**
said drive assembly (76) comprises a plurality of gears including a second gear (78) rotatably supported within said housing (12) by means of a hub (82), and that the anti-reverse mechanism comprises a roller clutch formed between said hub (82) and said second gear (78), said roller clutch being so arranged and constructed that movement of the trigger in the first direction engages actuation assembly (96) with drive assembly (76) to rotate the drive rod (30), while movement of the trigger in a second direction disengages actuation assembly (96) from drive assembly (76) to disable the actuation assembly (96) from rotating the drive rod (30).

2. The coil fastener applier of claim 1, wherein the interior of the elongated tubular portion (18) contains a surface (52) which engages a body portion (22) of the at least one coil fastener, the surface forcing the at least one coil fastener (20) distally as the drive rod is rotated in the first direction and wherein the drive rod (30) extends through a centre of the at least one coil fastener, the drive rod including a slot (32) for receipt of a tang portion (26) of a coil fastener.

3. The coil fastener applier of claim 1 or 2, wherein the drive assembly (76) includes a first bevelled gear (62) attached to the drive rod (30) and a second bevelled gear (78) engageable with the first bevelled gear and oriented perpendicularly to the first bevelled gear.

4. The coil fastener applier of any one of the preceding claims, wherein said anti-reverse mechanism includes a plurality of roller pins (160) positioned within a gap (162) defined between the second bevelled gear (78) and a hub (82), the roller pins engaging the second bevelled gear (with the hub when the roller pins are in a reduced area (166) of the gap and releasing the second bevelled gear from engagement with the hub when the roller pins are moved into enlarged areas (164) of the gap.

5. The coil fastener applier of any one of the preceding claims, wherein a plurality of coil fasters (20) are releasably mounted on the drive rod.

6. The coil fastener applier of any one of the preceding claims, wherein said actuation assembly (96) includes an idler gear (100) engageable with the drive assembly (76) and a ratchet-plate gear (98) engageable with the trigger (16), the idler gear being operatively associated with the ratchet-plate gear.

7. The coil fastener applier of claim 6, wherein the idler gear (100) is rotatably mounted with respect to the housing (12), the idler gear including first (142) and second (144) stops engageable with a blocking member (140) formed on the housing such that the degree of rotation of the idler gear is limited to that amount necessary to drive a single coil fastener (20) out of the elongated tubular portion (18) and
wherein the ratchet-plate gear (98) defines a slot (146) and the idler gear (100) includes a tab (152) projecting thereform and engageable with an edge (148) of the slot.

8. The coil fastener applier of claim 7, further comprising a compression spring (104) positioned within the slot (146), the compression spring urging the tab (152) into engagement with the edge (148) of the slot.

9. The coil fastener applier of any one of claims 6 to 8, further comprising a pawl (126) mounted on the housing (12) and engageable with ratchet teeth (124) formed on the ratchet-plate gear (98) and wherein the ratchet-plate gear includes a trigger gear (106) having teeth (114) engageable with teeth (112) formed on the trigger, such that pivoting the trigger (16) with respect to the housing (12) rotates the ratchet-plate gear (98) within a plane parallel to the longitudinal axis (x) of the drive rod (30).

10. The coil fastener applier of any one of the preceding claims, wherein each coil fastener includes a body portion (22) slideably mounted about the drive rod (30) and terminating in a tissue penetrating tip (24) at one end, the coil body portion (22) including a tang (26) at the opposite end, the tang engageable with slot (32) formed in the drive rod (30).

11. A coil fastener applier (200) according to any one of the preceding claims, wherein the at least one coil fastener (20) has a predetermined cross section, the cross section having a width diameter; and
wherein the distance (d) between a distalmost tip (226) of the drive rod (202) and the distalmost tip (212 of elongated tubular sleeve (204) of said elongated portion (206) is at least equal to half the predetermined width of the at least one coil fastener.

12. The coil fastener applier as recited in claim 11,
wherein a tang (26) of the at least one coil fastener (20) is retained within a slot (208) in the drive rod (202), the angle between the distalmost tip (222) of the threaded surface and the slot (208) when the trigger is in an unfired position is approximately 110°.

13. The coil fastener applier as recited in claim 11 or 12, further comprising a lockout mechanism engageable with the drive assembly to immobilise the drive rod (202) relative to the elongated tubular sleeve (204).

14. The coil fastener applier as recited in claim 13,
wherein the lockout mechanism includes a lockout button (236) movably mounted on the housing (232) and a blocking member (242) movable into engagement with the drive assembly in response to movement of the lockout button.

15. The coil fastener applier as recited in claim 14,
wherein the blocking member is a leaf spring (242) affixed at a first end (244) to the housing (232) and having a second end (248) engageable with the drive assembly, and wherein the lockout button includes a cam (240) engageable with the leaf spring to drive the second end (248) into engagement with the drive assembly in response to movement of the lockout button.

16. The coil fastener applier as recited in claim 15,
wherein the second end (248) of the leaf spring (242) includes a hook (246) engageable with teeth (250) on a gear (230) of the drive assembly, and wherein the blocking member pivots about a pivot pin in response to movement of the trigger.

## Patentansprüche

1. Spiralverschluss-Applikator (10) mit:
einem Gehäuse (12), das einen an dem Gehäuse angebrachten stationären Griff (14) besitzt;
einem länglichen, rohrförmigen Abschnitt (18), der sich in distaler Richtung von dem Gehäuse erstreckt;
einem Antriebsstab (30), der innerhalb des länglichen, rohrförmigen Abschnittes drehbar befestigt ist und mindestens einen Spiralverschluss (20), der an dem Antriebsstab lösbar befestigt ist, besitzt;
einer Antriebseinheit (76), die mindestens ein Zahnrad (62) umfasst, das zum Drehen des Antriebsstabs mit dem Antriebsstab (30) in Eingriff bringbar ist;
einer Betätigungseinheit (96), die mit der Antriebseinheit in Eingriff bringbar ist; und
einem Trigger (16), der an dem Gehäuse bewegbar befestigt ist und mit der Betätigungseinheit (96) in Eingriff bringbar ist, wobei eine Bewegung des Triggers in einer ersten Richtung den Antriebsstab (30) dreht, um einen Spiralverschluss (20) in distaler Richtung zu treiben;
bei dem die Antriebseinheit (76) ferner einen Antiumkehrmechanismus umfasst, um so eine Drehung des Antriebsstabs (30) in eine Richtung entgegengesetzt zu seiner Treibrichtung zu verhindern;
**dadurch gekennzeichnet , dass**
die Antriebseinheit (76) eine Vielzahl von Zahnrädern einschließlich eines zweiten Zahnrads (78), das innerhalb des Gehäuses (12) mit Hilfe einer Nabe (82) drehbar gelagert ist, aufweist, und dass der Antiumkehrmechanismus eine zwischen der Nabe (82) und dem zweiten Zahnrad (78) gebildete Rollenkupplung aufweist, wobei die Rollenkupplung derart angeordnet und aufgebaut ist, dass die Bewegung des Triggers in der ersten Richtung die Betätigungseinheit (96) in Eingriff mit der Antriebseinheit (76) bringt, um den Antriebsstab (30) zu drehen, während eine Bewegung des Triggers in eine zweite Richtung die Betätigungseinheit (96) von der Antriebseinheit (76) löst, so dass die Betätigungseinheit (96) den Antriebsstab (30) nicht mehr drehen kann.

2. Spiralverschluss-Applikator nach Anspruch 1, bei dem das Innere des länglichen, rohrförmigen Abschnitts (18) eine Oberfläche (52) enthält, die in Eingriff mit einem Korpusabschnitt (22) des mindestens einen Spiralverschlusses tritt, wobei die Oberfläche den mindestens einen Spiralverschluss (20) in distaler Richtung drängt, wenn der Antriebsstab in die erste Richtung gedreht wird, und bei der der Antriebsstab (30) durch eine Mitte des mindestens einen Spiralverschlusses verläuft, und der Antriebsstab einen Schlitz (32) zur Aufnahme eines Zungenabschnittes (26) eines Spiralverschlusses umfasst.

3. Spiralverschluss-Applikator nach Anspruch 1 oder 2, bei dem die Antriebseinheit (76) ein erstes kegelförmiges Zahnrad (62), das an dem Antriebsstab (30) angebracht ist, und ein zweites kegelförmiges Zahnrad (78), das mit dem ersten kegelförmigen Zahnrad in Eingriff bringbar ist und senkrecht zu dem ersten kegelförmigen Zahnrad ausgerichtet ist, umfasst.

4. Spiralverschluss-Applikator nach einem der vorhergehenden Ansprüche, bei dem der Antiumkehrmechanismus eine Vielzahl von Rollenstiften (160) umfasst, die innerhalb eines Spalts (162), der zwischen dem zweiten kegelförmigen Zahnrad (78) und einer Nabe (82) bestimmt ist, positioniert sind, wobei die Rollenstifte das zweite kegelförmige Zahnrad mit der Nabe in Eingriff bringen, wenn die Rollenstifte sich in einem reduzierten Bereich (166) des Spalts befinden, und das zweite kegelförmige Zahnrad aus dem Eingriff mit der Nabe gelöst wird, wenn die Rollenstifte in vergrößerte Bereiche (164) des Spalts bewegt werden.

5. Spiralverschluss-Applikator nach einem der vorhergehenden Ansprüche, bei dem eine Vielzahl von Spiralverschlüssen (20) auf dem Antriebsstab lösbar befestigt sind.

6. Spiralverschluss-Applikator nach einem der vorhergehenden Ansprüche, bei dem die Betätigungseinheit (96) ein Laufrad (100), das mit der Antriebseinheit (76) in Eingriff bringbar ist, und ein Ratschenzahnrad (98), das mit dem Trigger (16) in Eingriff bringbar ist, umfasst, wobei das Laufrad betriebsmäßig dem Ratschenzahnrad zugeordnet ist.

7. Spiralverschluss-Applikator nach Anspruch 6, bei dem das Laufrad (100) in Bezug auf das Gehäuse (12) drehbar befestigt ist und einen ersten (142) und einen zweiten (144) Anschlag umfasst, die mit einem Blockierelement (140) in Eingriff bringbar sind, das an dem Gehäuse derart gebildet ist, dass der Grad der Drehung des Laufrads auf einen solchen Grad begrenzt ist, der zum Heraustreiben eines einzelnen Spiralverschlusses (20) aus dem länglichen, rohrförmigen Abschnitt (18) notwendig ist, und wobei das Ratschenzahnrad (98) einen Schlitz (146) bestimmt, und das Laufrad (100) einen Vorsprung (152) umfasst, der hiervon hervorsteht und mit einer Kante (148) des Schlitzes in Eingriff bringbar ist.

8. Spiralverschluss-Applikator nach Anspruch 7, des weiteren mit einer Druckfeder, die den Vorsprung (152) in Eingriff mit der Kante (148) des Schlitzes drängt.

9. Spiralverschluss-Applikator nach einem der Ansprüche 6 bis 8, des weiteren mit einer Sperrklinke (126), die an dem Gehäuse (12) befestigt ist und mit den Ratschenzähnen (124), die auf dem Ratschenzahnrad (98) gebildet sind, in Eingriff bringbar sind, und bei dem das Ratschenzahnrad ein Triggerzahnrad (106) mit Zähnen (114) umfasst, die mit auf dem Trigger gebildeten Zähnen (112) in Eingriff bringbar sind, so dass eine Drehung des Triggers (16) in Bezug auf das Gehäuse (12) das Ratschenzahnrad (98) innerhalb einer Ebene parallel zu der Längsachse (X) des Antriebsstabs (30) dreht.

10. Spiralverschluss-Applikator nach einem der vorhergehenden Ansprüche, bei dem jeder Spiralverschluss einen Korpusabschnitt (22) umfasst, der um den Antriebsstab (30) verschiebbar befestigt ist und an einer Gewebedurchdringungsspitze (24) an einem Ende endet, wobei der Spiralkorpusabschnitt (22) eine Zunge (26) an dem gegenüberliegenden Ende umfasst, und die Zunge mit dem in dem Antriebsstab (30) gebildeten Schlitz (32) in Eingriff bringbar ist.

11. Spiralverschluss-Applikator (200) nach einem der vorhergehenden Ansprüche, bei dem der mindestens eine Spiralverschluss (20) einen vorbestimmten Querschnitt besitzt, und der Querschnitt einen breiten Durchmesser besitzt; und
bei dem der Abstand (d) zwischen einer am weitesten distal gelegenen Spitze (226) des Antriebsstabs (202) und der am weitesten distal gelegenen Spitze (212) der länglichen, rohrförmigen Hülse (204) des länglichen Abschnittes (206) mindestens gleich der Hälfte der vorbestimmten Breite des mindestens einen Spiralverschlusses ist.

12. Spiralverschluss-Applikator nach Anspruch 11, bei dem eine Zunge (26) des mindestens einen Spiralverschlusses (20) innerhalb eines Schlitzes (208) in dem Antriebsstab (202) gehalten ist, und der Winkel zwischen der am weitesten distal gelegenen Spitze (222) der Gewindeoberfläche und dem Schlitz (208) ungefähr 110° beträgt, falls der Trigger sich in einer nichtausgelösten Position befindet.

13. Spiralverschluss-Applikator nach Anspruch 11 oder 12, des weiteren mit einem Arretiermechanismus, der mit der Antriebseinheit in Eingriff bringbar ist, um den Antriebsstab (202) relativ zu der länglichen, rohrförmigen Hülse (204) zu immobilisieren.

14. Spiralverschluss-Applikator nach Anspruch 13, bei dem der Arretiermechanismus einen Arretierknopf (236), der auf dem Gehäuse (232) bewegbar befestigt ist, und ein Blockierelement (242), das in Eingriff mit der Antriebseinheit auf eine Bewegung des Arretierknopfes hin bewegbar ist, umfasst.

15. Spiralverschluss-Applikator nach Anspruch 14, bei dem das Blockierelement eine Blattfeder (242) ist, die an einem ersten Ende (244) an dem Gehäuse (232) angebracht ist, und ein zweites Ende (248) besitzt, das mit der Antriebseinheit in Eingriff bringbar ist, und bei dem der Arretierknopf eine Nocke (240) umfasst, die mit der Blattfeder in Eingriff bringbar ist, um das zweite Ende (248) in Eingriff mit der Antriebseinheit auf eine Bewegung des Arretierknopfes hin zu treiben.

16. Spiralverschluss-Applikator nach Anspruch 15, bei dem das zweite Ende (248) der Blattfeder (242) einen Haken (246) umfasst, der mit den Zähnen (250) auf einem Zahnrad (230) der Antriebseinheit in Eingriff bringbar ist, und bei dem das Blockierelement sich um einen Drehstift auf die Bewegung des Triggers hin dreht.

## Revendications

1. Applicateur d'agrafes en spirale (10) comprenant:
un boîtier (12) présentant une poignée stationnaire (14) fixée au boîtier;
une portion tubulaire oblongue (18) s'étendant distalement du boîtier;
une tige d'entraînement (30) montée à rotation dans la portion tubulaire oblongue et ayant au moins une agrafe en spirale (20) montée relâchablement sur la tige d'entraînement;
un ensemble d'entraînement (76) comprenant au moins une roue dentée (62) pouvant être mise en prise avec la tige d'entraînement (30) pour faire tourner la tige d'entraînement;
un ensemble d'actionnement (96) pouvant être mis en prise avec l'ensemble d'entraînement; et
une gachette (16) montée amoviblement sur le boîtier et pouvant être mise en prise avec l'ensemble d'actionnement (96), où le déplacement de la gachette dans une première direction fait tourner la tige d'entraînement (30) pour entraîner une agrafe en spirale (20) distalement;
où l'ensemble d'entraînement (76) comprend en outre un mécanisme anti-inverse de manière à empêcher la rotation de la tige d'entraînement (30) dans une direction opposée à celle de sa direction d'entraînement, **caractérisé en ce que**
ledit ensemble d'entraînement (76) comprend une pluralité de roues dentées incluant une deuxième roue dentée (78) supportée à rotation dans ledit boîtier (12) par un moyeu (82), et **en ce que** le mécanisme anti-inverse comprend un embrayage à rouleaux formé entre ledit moyeu (82) et ladite deuxième roue dentée (78), ledit embrayage à rouleaux étant agencé et construit de façon qu'un déplacement de la gachette dans la première direction mette en prise l'ensemble d'actionnement (96) avec l'ensemble d'entraînement (76) pour faire tourner la tige d'entraînement (30), tandis qu'un déplacement de la gachette dans une seconde direction sort l'ensemble d'actionnement (96) de prise avec l'ensemble d'entraînement (76) pour que l'ensemble d'actionnement (96) ne puisse pas faire tourner la tige d'entraînement (30).

2. Applicateur d'agrafes en spirale selon la revendication 1, où l'intérieur de la portion tubulaire oblongue (18) comprend une surface (52) qui vient en prise avec une portion de corps (22) d'au moins un applicateur d'agrafes précité, la surface forçant au moins un applicateur d'agrafes précité (20) distalement lorsque la tige d'entraînement est entraînée dans la première direction, et où la tige d'entraînement (30) s'étend à travers un centre d'au moins une agrafe en spirale précitée, la tige d'entraînement comprenant une fente (32) pour la réception d'une portion de queue (26) d'une agrafe en spirale.

3. Applicateur d'agrafes en spirale selon la revendication 1 ou 2, où l'ensemble d'entraînement (76) comprend un premier engrenage conique (62) fixé à la tige d'entraînement (30) et un deuxième engrenage conique (78) pouvant être mis en prise avec le premier engrenage conique et orienté perpendiculairement au premier engrenage conique.

4. Applicateur d'agrafes en spirale selon l'une des revendications précédentes, où ledit mécanisme anti-inverse comprend plusieurs axes de rouleau (160) positionnés dans un espace (162) défini entre le deuxième engrenage conique (78) et un moyeu (82), les axes de rouleau s'engageant dans le deuxième engrenage conique (avec le moyeu lorsque les axes de rouleau se trouvent dans une zone réduite (166) de l'espace et libérant le deuxième engrenage conique de la prise avec le moyeu lorsque les axes de rouleau sont déplacés dans des zones élargies (164) de l'espace.

5. Applicateur d'agrafes en spirale selon l'une des revendications précédentes, où plusieurs agrafes en spirale (20) sont montées relâchablement sur la tige d'entraînement.

6. Applicateur d'agrafes en spirale selon l'une des revendications précédentes, où ledit ensemble d'actionnement (96) comprend un engrenage intermédiaire (100) pouvant être mis en prise avec l'ensemble d'entraînement (76) et un engrenage de plaque à rochet (98) pouvant être mis en prise avec la gachette (16), l'engrenage intermédiaire étant fonctionnellement associé à l'engrenage de plaques à rochet.

7. Applicateur d'agrafes en spirale selon la revendication 6, où l'engrenage intermédiaire (100) est monté à rotation par rapport au boîtier (12), l'engrenage intermédiaire comprenant des premier (142) et second (144) arrêts pouvant être mis en prise avec un élément de blocage (140) formé sur le boîtier de sorte que le degré de rotation de l'engrenage intermédiaire est limité à cette quantité nécessaire pour entraîner une seule agrafe en spirale (20) hors de là portion tubulaire oblongue (18), et où l'engrenage de plaques à rochet (98) définit une fente (146), et l'engrenage intermédiaire (100) comprend une patte (152) faisant saillie de celui-ci et pouvant être mise en prise avec un bord (148) de la fente.

8. Applicateur d'agrafes en spirale selon la revendication 7, comprenant en outre un ressort de compression (104) positionné dans la fente (146), le ressort de compression sollicitant la pâte (152) en prise avec le bord (148) de la fente.

9. Applicateur d'agrafes en spirale selon l'une des revendications 6 à 8, comprenant en outre un cliquet d'arrêt (126) monté sur le boîtier (12) et pouvant être mis en prise avec les dents de rochet (124) formées sur l'engrenage de plaque à rochet (98), et où l'engrenage de plaque à rochet comprend un engrenage de gachette (106) ayant des dents (114) pouvant être mises en prise avec les dents (112) formées sur la gachette de sorte que le pivotement de la gachette (16) par rapport au boîtier (12) fait tourner l'engrenage de plaques à rochet (98) dans un plan parallèle à l'axe longitudinal (x) de la tige d'entraînement (30).

10. Applicateur d'agrafes en spirale selon l'une des revendications précédentes, où chaque agrafe en spirale comprend une portion de corps (22) montée d'une manière coulissante autour de la tige d'entraînement (30) et se terminant par une pointe (24) de pénétration dans le tissu à une extrémité, la portion de corps en spirale (22) comprenant une queue (26) à l'extrémité opposée, la queue pouvant s'engager dans la fente (32) ménagée dans la tige d'entraînement (30). ,

11. Applicateur d'agrafes en spirale (200) selon l'une des revendications précédentes, où au moins une agrafe en spirale précitée, (20) possède une section transversale prédéterminée, la section transversale ayant un diamètre en largeur; et
où la distance (d) entre la pointe la plus distale (226) de la tige d'entraînement (202) et la pointe la plus distale (212) du manchon tubulaire oblong (204) de ladite portion oblongue (206) est au moins égale à la moitié de la largeur prédéterminée d'au moins une agrafe en spirale précitée.

12. Applicateur d'agrafes en spirale selon la revendication 11, où une queue (26) d'au moins une agrafe en spirale précitée (20) est retenue dans une fente (208) dans la tige d'entraînement (202), l'angle entre la pointe la plus distale (222) de la surface filetée et la fente (208), lorsque la gachette se trouve dans une position non tirée, est approximativement de 110°.

13. Applicateur d'agrafes en spirale selon la revendication 11 ou 12, comprenant en outre un mécanisme de verrouillage pouvant être mis en prise avec l'ensemble d'entraînement afin d'immobiliser la tige d'entraînement (202) relativement au manchon tubulaire oblong (204).

14. Applicateur d'agrafes en spirale selon la revendication 13, où le mécanisme de verrouillage comprend un bouton de verrouillage (236) monté d'une manière mobile sur le boîtier (232) et un élément de blocage (242) pouvant être amené en prise avec l'ensemble d'entraînement en réponse à un mouvement du bouton de verrouillage.

15. Applicateur d'agrafes en spirale selon la revendication 14, où l'élément de blocage est un ressort à lames (242) fixé à une première extrémité (244) au boîtier (232) et ayant une seconde extrémité (248) pouvant être mise en prise avec l'ensemble d'entraînement, et où le bouton de verrouillage comprend une came (240) pouvant être mise en prise avec le ressort à lames pour entraîner la seconde extrémité (248) en prise avec l'ensemble d'entraînement en réponse à un mouvement du bouton de verrouillage.

16. Applicateur d'agrafes en spirale selon la revendication 15, où la seconde extrémité (248) du ressort à lames (242) comprend un crochet (246) pouvant être mis en prise avec des dents (250) sur une roue dentée (230) de l'ensemble d'entraînement, et où l'élément de blocage pivote autour d'un pivot en réponse au mouvement de la gachette.
